# EUROPEAN PATENT SPECIFICATION

(11) **EP 1 480 510 B1**
(45) Date of publication and mention of the grant of the patent: **19.11.2008**
(21) Application number: 03712646.3
(22) Date of filing: 03.03.2003
(51) Int. Cl.: A01H 5/00, A01H 5/10, C12N 15/82, C12N 9/24

(54) **EXPRESSION OF LYSOSOMAL ENZYMES IN PLANT SEEDS**
EXPRESSION VON LYSOSOMALEN ENZYMEN IN PFLANZENSAMEN
EXPRESSION D'ENZYMES LYSOSOMALES DANS DES SEMENCES

(30) Priority: 01.03.2002 IT RM20020115
(43) Date of publication of application: 01.12.2004
(73) Proprietor: Plantechno SRL, 26040 Vicomoscano (IT)
(72) Inventor: FOGHER, Corrado, I-26041 Casalmaggiore (IT); REGGI, Serena, I-29100 Piacenza (IT)
(74) Representative: Predazzi, Valentina
(86) International application number: PCT/IT2003/000120
(87) International publication number: WO 2003/073839

(56) References cited:
- WO-A-00/04146
- WO-A-02/08404
- WO-A-97/10353
- WO-A-99/16890
- US-B1- 6 288 304

## Description

### Field of the invention

The present invention relates to the production of transgenic plants able to express, in seed storage tissues, a lysosomal enzyme in enzymatically active form and in amounts appropriate to its use in enzyme replacement therapy.

In particular, the present invention relates to plants and seeds containing this enzyme.

### Prior state of the art

Lysosomal diseases represent a wide class of human genetic diseases determined by malfunctions of specific lysosomal enzymes. Lysosomes are the main degradative organelles of animal cells and are of critical importance in degrading macromolecules and in recycling their monomeric components. These membrane-delimited organelles exhibit acidic pH and contain a variety of nucleases, proteases, phosphatases and degradative enzymes for polysaccharides, mucopolysaccharides and lipids. A malfunction of a specific acid hydrolase determines an aberrant accumulation of the substrate in the lysosomes, causing a variety of pathologies, among which there may be mentioned: Tay-Sachs disease, due to a deficiency of the enzyme β-N-hexosaminidase, Mucopolysaccharidoses (MPSs) a group of recessive disorders due to a malfunction in the degradation of complex sulphurates, Anderson-Fabry disease, due to a deficiency of the enzyme α-galactosidase A causing accumulation of globotriaosylceramide mostly in renal microvascular endothelial cells, Pompe disease, due to a deficiency of the enzyme acid α-glucosidase leading to intralysosomal accumulation of glycogen and Gaucher disease, due to deficiences in the enzyme glucocerebrosidase (acid β-glucosidase or GCB) that determines the accumulation of glycosphingolipids mainly in cells of monocyte-macrophage lines.

In particular, Anderson-Fabry disease is a dominant X-linked disease resulting from the malfunction of the enzyme α-galactosidase A (α-Ga1A or GLA), which leads to the progressive accumulation of globotriaosylceramide (GL-3) and of the related glycosphingolipids causing proteinuria in young males and subsequently, with age, kidney failure.

Human α-galactosidase A is a 429 amino acids (aa) homodimeric glycoprotein, with four putative glycosylation sites, whose signal peptide is represented by the 31 N-terminal residues. The cDNA sequence of GLA has been published by Tsuji S. et al., 1987, Eur. J. Biochem., 165(2), 275-280.

Pompe disease, or glycogen storage disease type II, is an autosomal recessive metabolic myopathy caused by a deficiency of the enzyme acid β-glucosidase (GAA) which leads to storage of glycogen in almost all tissues, specifically injuring heart and skeletal muscles.

Acid β-glucosidase is a 952 aa glycoprotein having seven putative glycosylation sites, whose signal peptide is represented by the 69 N-terminal residues. cDNA sequence has been published by Hoefsloot L. H. et al., 1988, EMBO JOURNAL 7(6), 1697-1704.

Lastly, Gaucher disease is an autosomal recessive disorder caused by a deficiency of glucocerebrosidase, an enzyme required for degradation at lysosomal level of lipids containing covalently bonded sugars (Brady et al. 1965, J. Biol. Chem., 240: 39-43). In the absence of glucocerebrosidase, the insoluble compound glucocerebroside (glucosylceramide) accumulates in the lysosomes leading to the disease symptomatology.

Human glucocerebrosidase is a glycoprotein having a molecular weight ranging from 58 to 70 kDa, with five putative glycosylation sites. The complete cDNA has been reported (Sorge et al., 1985, Proc. Natl. Acad. Sci. 82: 7289-7293). The open reading frame, having 1545 base pairs (bp) plus several introns, codes for a protein having 515-amino acid, the 19 amino acids of the peptide signal included.

Success attained by enzyme replacement therapy in lysosomal diseases like Gaucher disease underlined the demand for more effective, safe and economical production methods of lysosomal enzymes in general.

To date, some of these enzymes have been directly extracted from human placenta or produced, by genetic engineering, in CHO (Chinese hamster ovary) cell cultures. Both of the abovementioned production methods, besides being very costly, entail risks for the patient. In fact, following the use of placenta-extracted enzymes, entailed viral infections have been observed, whereas following the use of enzymes extracted from CHO cell cultures, in the 15% of the patients production of specific antibodies against the product was detected, whose origin could relate to the production system adopted.

Only recently some lysosomal enzymes have been produced in genetically modified plants.

Human lysosomal enzymes can be produced in transgenic plants in order to solve problems of safety, viral infections, immune reactions, production yield and cost.

U.S. Patent 5,929,304 reports the "in-leaf" production of some lysosomal enzymes (human glucocerebrosidase and human α-L-iduronidase), optimally generated in tobacco.

In fact, tobacco is particularly indicated as a model system for the production of high-value recombinant proteins. Heterologous genes can be introduced in totipotent cells of tobacco using nonpathogenic strains of *Agrobacterium tumefaciens.* The subsequent growth and differentiation of the transformed cells leads to the attainment of stable transgenic plants. Large amounts of leaf material from transgenic plants are yielded in 6-7 weeks, whereas first-generation transgenic seeds, in amounts vastly greater than those of the leaf material, become available in other 2-3 months.

U.S. Patent 5,929,304 describes the expression of some lysosomal enzymes in tobacco plants. The enzymes are produced essentially in leaf by plants transformed via the use of vectors containing the MeGa promoter (deriving from the tomato HMG2 promoter) or the cauliflower mosaic virus (CaMV) 35S promoter. U.S. Patent 5,929,304 reports, among the promoters indicated as useful in carrying out the invention, besides the abovecited ones, also the rbcS promoter, the chlorophyll-binding a/b protein, the AdhI promoter and the NOS promoter. According to the teachings of this latter patent, the promoter used may be constitutive or inducible. In-leaf expression of enzymatically active human □-L-iduronidase is described in all the examples reported in the patent. The expression occurs following the transformation of tobacco plants with vectors containing, besides the sequence coding for the desired enzyme, the mechanically inducible MeGA promoter, or the constitutive 35S promoter, respectively.

As reported in the examples, the yield of human glucocerebrosidase in tobacco leaves is equal to about 1.5 mg of extractable protein per 1000 mg of tissue, i.e. the yielded enzyme is equal to the 0.15% b/w of the initial leaf material. This result, obtained by in-plant expression, has been indicated by the inventors as the long-sought solution for the production of animal or human lysosomal enzymes, thereby meeting the demand for said enzymes in replacement therapy.

However, in the leaf protein concentration is known to be extremely low, and anyhow lower than the one in seed

Moreover, it has to be borne in mind that in the case of in leaf enzyme expression there subsists a stability problem, due to the fact that enzymes are proteins extremely sensible to denaturating factors. Said stability problem requires the continual and prompt harvesting of the leaf tissue after the mechanical induction of the promoter, the immediate freezing at -20°C of said tissue and the discarding of an enormous amount of material in the enzyme-extracting step. Moreover, the in-leaf expression allows production of a very low amount of proteins, anyhow lower with respect to an in seed production of the same. Hence, it would be useful to provide a seed-specific system for the expression of lysosomal enzymes.

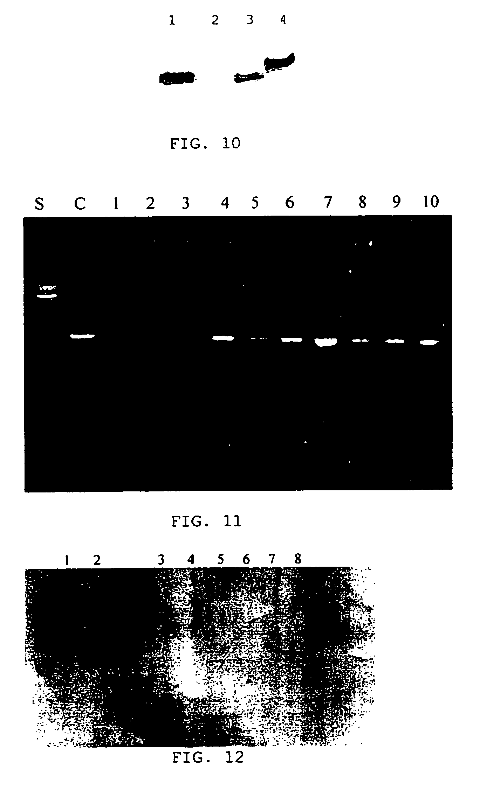

Although the teachings of U.S Patent 5,929,304 theoretically relate also to the expression of the enzyme in plant tissues other than the leaf one, it has been verified by the present Inventors, while attempting at expressing acid β-glucosidase in seed, that the 35S promoter identified in said patent as one of the promoters suitable for this purpose is unable to direct in-seed accumulation of a stable protein (Figure 12). As it is subsequently reported in the comparative example, tobacco plants were transformed using an expression vector comprising the cDNA sequence of the human glucocerebrosidase gene, coding also for the native signal sequence of the enzyme, under the control of the 35S promoter. Western Blot analysis under chemiluminescence demonstrated the absence of the desired enzyme from seed tissues. Hence, U.S patent 5,929,304, although declaring the described method as suitable for in-seed expression, does not provide the teachings required in order to carry out said expression to a person skilled in the art.

### In seed expression of heterologous proteins

Overall, seed constitutes the vegetal organ most widely used by humans for its caloric and protein contributions. Storage function for the nitrogen component is carried out by specific proteins accumulated in protein bodies, inside compartments in endocellular membrane systems. In seed, protein amounts range from about the 10-15% b/w in cereals to about the 25-35% in Leguminosae, whereas in tobacco they are equal to about the 20% b/w. Hence, protein accumulation should be directed in seed endosperm in order to increase the production yields of in plant expressed proteins.

International Patent Application WO-A-00/04146, to the same applicant, described the in-seed expression of protein lactoferrin. This result was obtained by modifying tobacco and rice plants with vectors containing an expression cassette. The cassette comprises a promoter, and a DNA sequence coding for the signal sequence of storage proteins highly abundant in seeds and stage-specifically expressed, i.e. βconglycinine or basic S7 globulin, and a DNA sequence coding for lactoferrin. WO-A-00/04146 details the vectors used and the promoters selected.

Lactoferrin is a glycoprotein able to bind iron and usually expressed in human milk. This protein exhibits an extremely high stability, given that lactoferrin-containing solutions may be treated even for 5 min at 90°C without detecting significant losses in protein activity. Pat. Appln. WO 00/04146 advances, by way of hypothesis, also the expression of proteins exhibiting a generically enzymatic activity using the method practiced with lactoferrin. However, this possibility remains purely theoretical, as no data is provided supporting the validity of the system adopted in said Patent Application for the expression of functionally active enzymes, even less so of lysosomal enzymes.

Pat. Appln. WO-A-00/04146, it being actually not aimed to enzyme expression, neglects several problems of fundamental relevance related to the stability of the hexogenous protein expressed. In fact, the utmost stability of lactoferrin and the fact that in lactoferrin the presence of non-natural glycosidic chains does not influence protein folding and function, definitely bars the use of the system disclosed in Pat. Appln. WO-A-00/04146 from effectively expressing enzymes exhibiting a correct folding and being functionally equivalent to the native enzyme. Moreover, it is known that in the case of the acid □-glucosidase the glycosylation of the first of the five glycosylation sites in the enzyme is crucial to the generation of an active enzyme; said function pertains to the third glycosylation site in the case of □ galactosidase A, and in the case of acid β-glucosidase, the second glycosylation site is the one crucial to the generation of the mature enzyme.

Another problem lies in that the protein instability could be due to structural restraints, but also be a consequence of its subcellular location. For the expression of lysosomal enzymes the fact should be taken into account that plant cells do not possess lysosomes, using the vacuole as a functional analogue of these acid vesicles for polymer degradation. In humans, the main pathway for transferring soluble enzymes to lysosomes implies the interaction with mannose-6-phosphate receptors (MPR). Lysosomal proteins are dispatched to the endoplasmic reticulum (RE) by N-terminal signal peptides, and are glycosilated during their transfer to Golgi apparatus. In this organelle, the N-linked glycans of the enzymes destinated to the lysosomes are selectively phosphorylated to one or more mannose residues. Then, the mannose-6-phosphate can bind to the membrane receptors which ensure glycoprotein internalization.

Plants lack the targeting system based on mannose-6-phosphate, do not possess MPRs and do not apppear to produce phosphorylated glycans. Since lysosomal enzymes function in an acidic environment (pH <4) and are unstable at higher (≥7) pH values, the pH (5.8) of the plant (extracellular) apoplastic compartment makes the secretion of said enzymes in said compartment ideal for their stability. Although it is inferrable that human lysosomal glycoproteins may be secreted in the apoplastic space (which would be particularly suitable for the storage of lysosomal enzymes), it does not follow that the system disclosed in the abovecited Patent Application be able to vehicle lysosomal enzymes in the apoplastic space of the seed storage tissue. For the abovementioned reasons, this localization could provide stability to expressed enzymes; in fact, an accurate localization of the lysosomal enzymes inside plant tissues is of fundamental importance for the stability of the produced enzyme.

Moreover, as it is known, unlike lactoferrin, enzymes are in most cases extremely unstable proteins requiring low-temperature storage, being easily inactivated by heat or denaturating agents.

In the light of these open questions, a person skilled in the art would hardly find and reckon in WO-A-00/04146 the technical support required to successfully express active lysosomal enzymes in storage organs of plant seeds.

Object of the present invention is to solve the problems left unsolved by the abovementioned state of the art. In particular, object of the present invention is to produce an expression system allowing the generation of transgenic plants able to express in seed a lysosomal enzyme being stable and in an enzymatically active form. A further object of the present invention is to produce such enzyme in an amount greater than that obtainable in leaf, i.e. greater than 1.5 mg per gram of tissue used.

### Summary of the Invention

The invention is based on the unexpected discovery that lysosomal enzymes, of animal or human origin, can be advantageously expressed in seed storage organs in a form which is stable (stability exceeds 12 months in appropriately stored seeds), enzymatically active and in a high amount suitable for a medical use of said enzymes.

This amount is not lower than the 0.8%, preferably than the 1%. In an optimal form, the yield is of about the 1.5% of the total seed proteins, in order to use said seeds as storage and preservation means of such enzyme.

Moreover, regardless from the amount of expressed enzyme, the effective storage of the enzyme over lenghty periods (>12 months) enables to plan production at more favourable season periods, something not viable with in-leaf production, which requires immediate extraction and purification of the enzyme.

Within the present description, with the term "enzymatically active form" it is meant that the enzyme is functionally active and that its activity is equivalent to that of the native enzyme.

Hence, object of the present invention is a genetically transformed plant able to produce a lysosomal enzyme of animal or human origin, characterised in that:
- said plant is transformed via the use of a recombinant expression vector comprising:
   a. a promoter of a plant gene specific for the expression in seed storage organs and stage-specific;
   b. a DNA sequence encoding the signal sequence of a plant protein able to dispatch said lysosomal enzyme to seed storage organs and to provide the post-translational modifications required for the expression of the enzyme in active form;
   c. a DNA sequence encoding said lysosomal enzyme deleted of the sequences encoding the signal sequence of the native enzyme and the sequence encoding the native polyadenylation signal;
   d. and a polyadenylation signal;
- said enzyme is expressed in seed storage tissues in enzymatically active form and in an amount of at least the 0.8%, preferably of the 1%, of the total proteins of the seed.

Objects of the present invention are also the method for producing said plant by transforming plant cells with the abovedescribed vector and by regenerating plants from said transformed cells, the seeds produced by said plant, the method for producing said seeds, the method for purifying the enzyme from said seeds, the use of said seeds for the preparation of medicaments for enzyme replacement therapy and the use of said seeds as means for storing and preserving a lysosomal enzyme in enzymatically active form.

### Detailed description of the figures

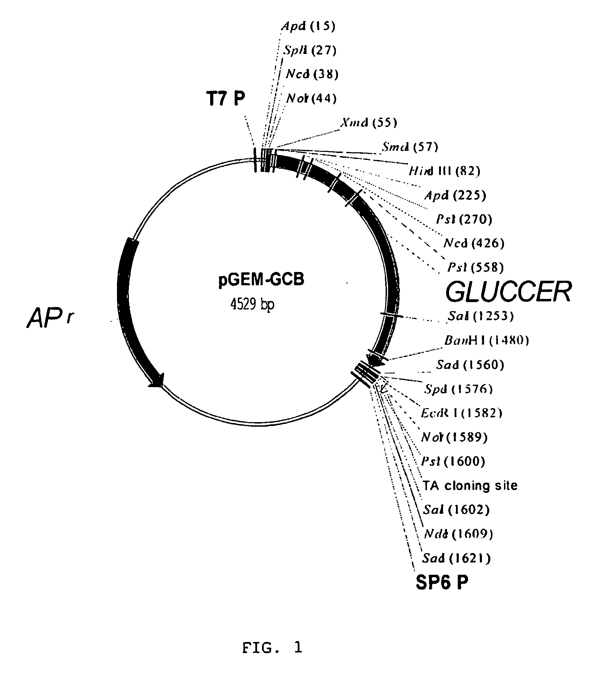

Figure 1 shows the plasmid named pGEM-GCB obtained from the initial cloning of the GCB gene and used for the control of the complete sequence of the amplified gene. pGEM-GCB is obtained by cloning the fragment corresponding to the GCB gene having sequence SEQ ID NO 1 (obtained by cloning total RNA from human placenta by RT-PCR (reverse transcriptase PCR) with the primers having SEQ ID NO 3 and 4) amplified with the primers having SEQ ID NO 4 and 5 in pGEM®-T plasmid (Promega) in the EcoRV linearization site, and then delimiting the GCB gene between SacI and SmaI sites, in primers having SEQ ID NO 5 and 4, respectively. The primer having SEQ ID NO 5 allows the deletion of the DNA sequence portion encoding the human signal sequence from SEQ ID NO 1.

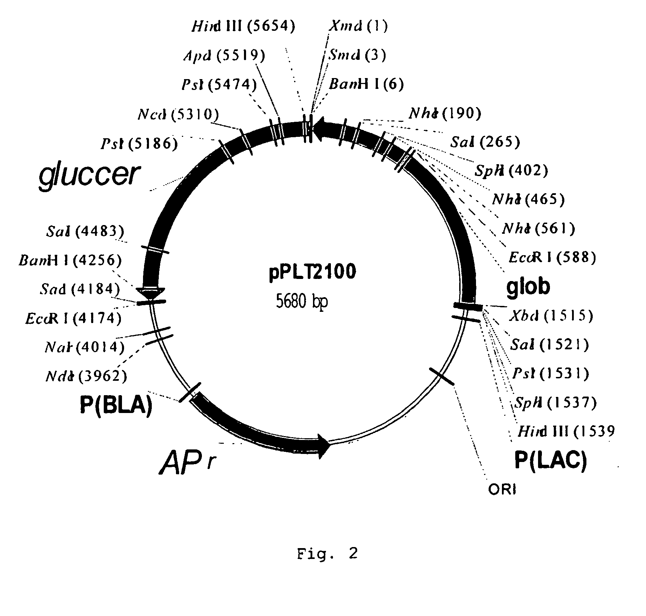

Figure 2 shows the plasmid named pPLT2100, used to construct the PGLOB-GCB chimeric gene. pPLT2100 is obtained by inserting, between the SmaI and SacI sites of the pUC19 vector polylinker (EMBL Acc. N. X02514), the fragment corresponding to the GCB gene cloned in the plasmid of Figure 1 cleaved with the same enzymes and the PGLOB promoter (SEQ ID NO 6) plus the sequence coding for the signal sequence of basic soy globulin (SEQ ID NO 7) cloned between the XbaI and BamHI sites.

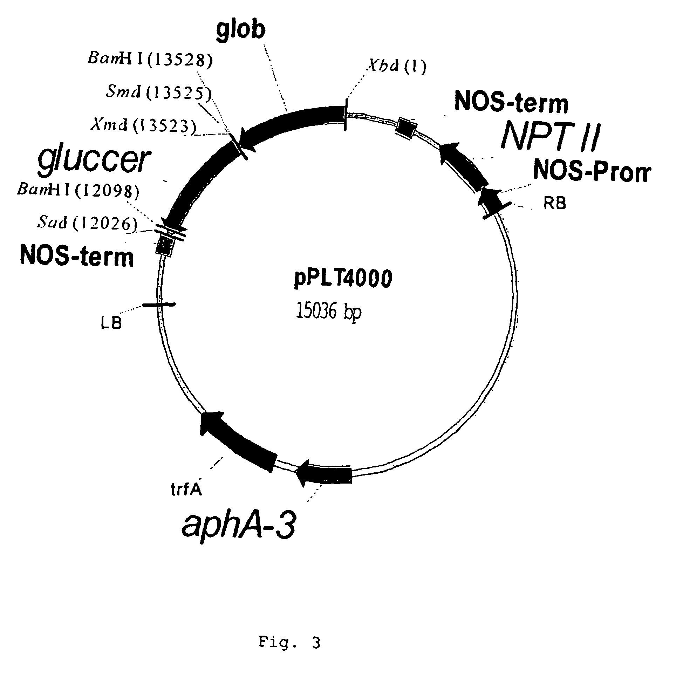

Figure 3 shows the plasmid named pPLT4000 obtained by inserting between the XbaI-SacI sites of the pBI101 plasmid (Clontech) the PGLOB-GCB cassette cleaved with the same enzymes (XbaI-SacI) from the vector of Figure 2.

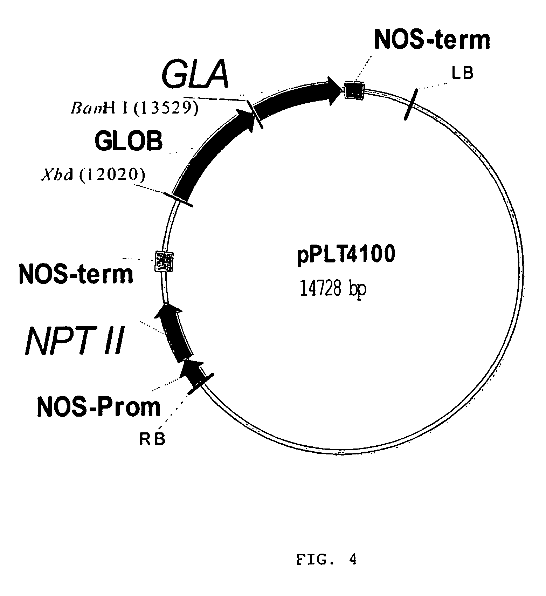

Figure 4 shows the plasmid named pPLT4100 obtained by inserting between the BamHI-SacI (blunt) sites of the pBI101 plasmid (Clontech) the PGLOB-GLA cassette comprising the PGLOB promoter (SEQ ID NO 6), the signal sequence of the basic 7S soy globulin (SEQ ID NO 7) and the cDNA of the human □ galactosidase A gene (SEQ ID NO 8), indicated as GLA, deleted of the nucleotides coding for the signal peptide (i.e., deleted up to nucleotide 116). The PGLOB-GLA cassette was obtained by constructing plasmids analogous to those shown in figures 1 and 2 in which the cDNA cloned is that of human α-galactosidase A (SEQ ID NO 8) deleted of the nucleotides encoding the signal peptide using the primers having sequences SEQ ID NO 10 and SEQ ID NO 11, which add a BamHI restriction site at 5', and the ECORV (blunt) restriction site at 3' of the amplified DNA, respectively.

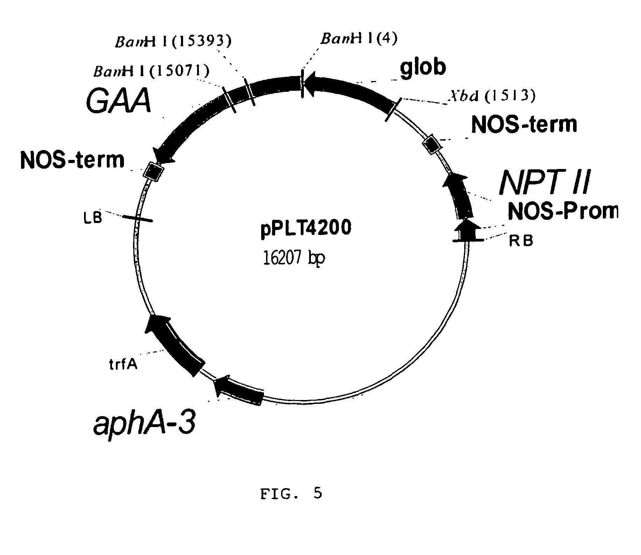

Figure 5 shows the plasmid named pPLT4200 obtained by inserting between the SmaI-SacI sites (SacI is cleaved with the blunt EcoCRI isoschizometer) of the pBI101 plasmid (Clontech) the PGLOB-GAA cassette comprising the PGLOB promoter (SEQ ID NO 6), the signal sequence of the basic 7S soy globulin (SEQ ID NO 7) and the cDNA of the human acid α-glucosidase gene (SEQ ID NO 12) indicated as GAA deleted of the nucleotides encoding the signal sequence (i.e. deleted up to nucleotide 426). The PGLOB-GAA cassette was obtained by constructing plasmids analogous to those shown in figures 1 and 2 in which the cloned DNA is that of human acid α-glucosidase (SEQ ID NO 12), deleted of the nucleotides coding for the signal peptide using the primers having SEQ ID 14 and SEQ ID 15, which add the EcoRV (blunt) restriction site at 5' and at 3' of the amplified DNA.

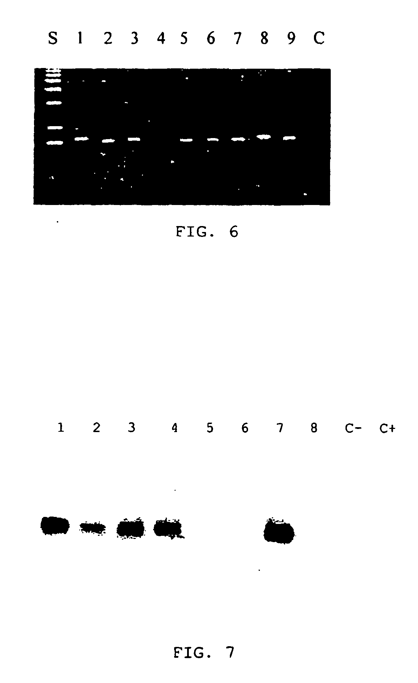

Figure 6 reproduces the result of an agarose gel electrophoresis of the DNA from PCR amplification carried out with primers specific for the human glucocerebrosidase gene (SEQ ID NO 4 and 5), on DNA extracted from plants transformed with the construct of Figure 3. The amplified fragment of about 1500 base pairs (bp) corresponds to the human glucocerebrosidase gene transformed in tobacco and present in the genome of nearly all the kanamycin-resistant tobacco lines obtained. The molecular weight marker ladder: 1Kb ladder (Promega) (bands from bottom to top 1000bp, 1500bp, 2000bp, 3000bp, 4000bp, 5000bp, 6000bp and 7000bp) was loaded in the well of lane S. The product of DNA amplification of nine transformed tobacco plants was loaded in the wells of lanes 1 to 9. The product of the amplification carried out on the DNA of wild tobacco plants, not containing a homologous sequence amplifiable with the same primers, was loaded in the well of the C lane.

Figure 7 reports the results of Northern analysis performed on RNA extracted from immature seeds (Days After Pollination, DAP, 20) of tobacco lines, transformed with the plasmid of Figure 3 and tested positive at PCR control for gene presence detection, reported in Figure 6. Total RNA extracted with the Trizol® method was loaded (10 µg/well) on agarose gel, subjected to electrophoresis, transferred on a nylon membrane and hybridised with a radioactive probe obtained by amplifying a human glucocerebrosidase gene fragment. Total RNA extracted from transgenic plants was loaded in lanes 1 to 8; C- is the negative control consisting of total RNA of a wild-type tobacco plant, C+ is the positive control consisting of total RNA (15□g) extracted from human placenta. The dimensions of the underlined band correspond to those expected for the messenger RNA (mRNA) of the GCB gene. The gene is not transcribed in plants 5, 6 and 8.

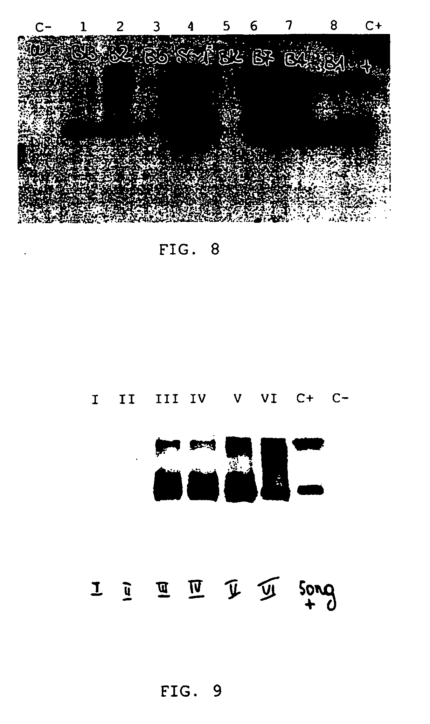

Figure 8 reports a SDS-PAGE gel of proteins partially purified from seeds of transgenic tobacco plants transformed with the plasmid of Figure 3 (lanes 1 to 8) reported in Figures 6 and 7, and their Western analysis. Western analysis was carried out on the total raw protein extract (1 mg) of mature seed, separated by SDS-PAGE electrophoresis. The analysis was carried out with chemiluminescence techniques, using a rabbit polyclonal antibody specific for human glucocerebrosidase enzyme as primary antibody, and a peroxidase-conjugated anti-rabbit IgG as secondary antibody. C- indicates the negative control, consisting of proteins extracted from wild-type tobacco seed; C+ indicates the positive control, consisting of commercial human glucocerebrosidase enzyme (50ng).

Figure 9 reports the expression profile of the PGLOB promoter in the tobacco seed of a transgenic line, regulating the expression of the GCB gene in the more productive lines. The different lanes contain total protein extracts obtained from the seed of a same line harvested at subsequent times after fertilization (DAP: day after pollination). Western Blot analysis, following the same procedure indicated for Figure 8, ascertained the presence of the protein corresponding to the human glucocerebrosidase enzyme. Lane I: DAP 4; lane II: DAP 9; lane III: DAP 14; lane IV: DAP 18; lane V: DAP 22; lane VI: complete maturation; lane C+: positive control, commercial product 50 ng; lane C-: negative control, total protein extract from wild-type tobacco plants.

The results shown in this Figure highlight that in tobacco the soy PGLOB promoter has an activation profile which is optimal for the production of recombinant proteins, it being activated at about DAP 10 and expressed until maturation.

Figure 10 reports the results of the deglycosilation enzyme treatment of the glucocerebrosidase protein purified from tobacco seed. Lane I: deglycosilated commercial human glucocerebrosidase enzyme, lane 2: control with proteins extracted from wild-type tobacco seed; lane 3: enzyme purified from seed and deglycosilated by deglycosilation enzyme treatment; lane 4: enzyme purified from seeds and not subjected to deglycosilation.

Figure 11 reproduces the result of an agarose gel electrophoresis of the DNA resulting from PCR amplification, carried out with primers specific for the human α-galactosidase A gene (SEQ ID NO 10 and 11), on DNA extracted from plants transformed with the construct of Figure 4. The amplified fragment of about 1200 bp corresponds to the human α-galactosidase A gene transformed in tobacco and present in the genome of nearly all the kanamycin-resistant tobacco lines obtained. The molecular weight marker ladder: 1Kb ladder (Promega) (bands bottom to top 1000bp, 1500bp, 2000bp, 3000bp and 4000bp) was loaded in the well of lane S. The product from DNA amplification of nine transformed tobacco plants was loaded in the wells of lanes lane 2-11. The product of the amplification carried out on the plasmid used for the transformation was loaded in the well of lane C.

Figure 12. Western Blot related to the in-seed expression control of two tobacco lines transformed with GCB under control of the 35S promoter, and to the DNA sequence coding for the signal sequence of the human GCB gene. Western analysis was carried out with chemiluminescence techniques, using a rabbit polyclonal antibody specific for human glucocerebrosidase enzyme as primary antibody, and a peroxidase-conjugated anti-rabbit IgG as secondary antibody.

Lane 1: human glucocerebrosidase (50 ng), purified from placenta and deglycosilated; lane 2: human glucocerebrosidase (50 ng), non-deglycosilated and purified from placenta; lane 3: total proteins (1 mg) extracted from SR-S9 line seed; lane 4: total proteins (1 mg) extracted from SR-S10 line seed; lane 5: total proteins (1 mg) extracted from SR-S11 line seed; lane 6: total proteins (1 mg) extracted from SR-S12 line seed; lane 7: total proteins (1 mg) extracted from SR-S13 line seed; lane 8: total proteins (1 mg) extracted from SR-S14 line seed.

### Detailed description of the Invention

The lysosomal enzymes according to the present invention include but are not limited to: α-N-acetylgalactosaminidase, acid lipase, aryl sulfatase A, aspartylglycosaminidase, ceramidase, α-fucosidase, α-galactosidase A, α-galactosidase, galactosylceramidase, glucocerebrosidase, α-glucosidase, β-glucuronidase, heparin N-sulfatase, β-hexosaminidase, iduronate sulfatase, α-L-iduronidase, α-mannosidase, -mannosidase, sialidase and sphingomyelinase.

A person skilled in the art will know said enzymes, as well as their nucleotide and polypeptide sequences. The sequence encoding said enzymes might be isolated in accordance with any one known method. A preferred method for isolating said sequences is based on the RT-PCR technique. Suitable primers (including or not including the DNA sequence encoding the native signal sequence of the protein and the polyadenylation signal) may be designed from known sequences, and the cDNA of the desired gene may be cloned by RT-PCR from mRNA extracted from placental cells.

The cloned sequences including the DNA encoding the native signal sequence and/or the polyadenylation signal may subsequently be modified, by enzymatic treatments or by further PCR amplification using suitable primers, so as to delete said sequences.

In a preferred embodiment of the present invention, the lysosomal enzyme is human acid β-glucosidase (glucocerebrosidase or GCB). The cDNA of said enzyme (SEQ ID NO 1) may be cloned from mRNA extracted from placental cells using the primers having SEQ ID NO 3 and 4. Said cDNA, containing the sequence coding for the GCB native signal sequence, may further be PCR-amplified using the primers having SEQ ID NO 4 and 5, which delete said sequence, i.e. nucleotides 1-57 of SEQ ID NO 1, and adding restriction sites suitable for an easier insertion of the amplified product in the recombinant expression vector. The polyadenylation sequence is already absent from SEQ ID NO 1.

In another preferred embodiment of the present invention, the lysosomal enzyme is human α-galactosidase A (α-GalA or GLA). The cDNA of said enzyme (SEQ ID NO 8) already deleted of the portion encoding the signal peptide (i.e. to nucleotide 116), may be cloned from mRNA extracted from placental cells using the primers having SEQ ID NO 10 and 11. Said primers add restriction sites suitable for an easier insertion of the amplified product in the recombinant expression vector. The polyadenylation sequence is already absent from SEQ ID NO 8.

In yet another preferred embodiment of the present invention the lysosomal enzyme is human acid α-glucosidase (GAA). The cDNA of said enzyme (SEQ ID NO 12) already deleted of the portion encoding the signal peptide (i.e., to nucleotide 426), may be cloned from mRNA extracted from placental cells using the primers having SEQ ID NO 14 and 15. Said primers add restriction sites suitable for an easier insertion of the amplified product in the recombinant expression vector. The polyadenylation sequence is already absent in SEQ ID NO 12.

The recombinant expression vector for plant transformation of the present invention may be any one known vector suitable for the transformation of plant cells and for the expression of protein products in them. Said vector may be cleaved at the most appropriate restriction sites, and in it, an expression cassette according to the present invention may be inserted.

Suitable plant transformation vectors according to the present invention include, but are not limited to, *Agrobacterium* Ti plasmids and derivatives thereof, including both integrative and binary vectors, plasmids pBIB-KAN, pGA471, pEND4K, pGV3850, pMON505 and pBI101. Included among the vectors of the present invention are also DNA or RNA plant viruses like, e.g., cauliflower mosaic virus, tobacco mosaic virus and their engineered derivatives genetically suitable for the expression of lysosomal enzymes. Moreover, transposable elements may be used in conjunction with any vector to transfer the expression cassette of the present invention into the plant cell.

Preferred vector for the purposes of the present invention is the expression plasmid pBI101 in which an expression cassette is inserted.

The expression cassette according to the present invention comprises a promoter of a plant gene, stage-specific and specific for the expression in seed storage organs. As stage-specific, it is meant a promoter inducing the expression of the gene it controls at a specific seed development stage.

A promoter suitable for practicing of the present invention is the promoter of the basic 7S soy globulin (SEQ ID NO 6). The expression cassette according to the present invention further comprises a DNA sequence coding for the signal sequence of a plant protein able to dispatch said lysosomal enzyme to the seed storage organs and to ensure the post-translational modifications required for the expression of the enzyme in enzymatically active form.

A signal sequence suitable for practicing the present invention is the signal sequence of basic 7S soy globulin (SEQ ID NO 7).

The expression cassette according to the present invention also contains a DNA sequence encoding a lysosomal enzyme of the ones listed above, deleted of the sequences encoding the native signal sequence and the polyadenylation signal.

Moreover, the expression cassette of the present invention contains a polyadenylation signal (or that already present in the expression vector can be used).

The elements constituting the expression cassette of the present invention should be functionally linked, in the aboveindicated order in the 5'-> 3' direction.

Optionally, the nucleotide sequence encoding the enzyme may be preceded by a short sequence apt to ease purification of the same protein on affinity columns, like e.g., his 6x tag, FLAG® (Sigma) or GST (Amersham).

In a preferred embodiment of the present invention, the vector derives from pBI101 plasmid (Clontech) in which the abovedescribed expression cassette is inserted. Said cassette comprises, besides the DNA encoding the desired lysosomal enzyme, the promoter of the basic 7S soy globulin gene (PGLOB) (SEQ ID NO 6) and the DNA coding for the signal sequence of the gene of the basic 7S soy globulin (SEQ ID NO 7). Said expression cassette, inserted in pBI101 vector (Clontech) upstream of the polyadenylation signal already present in the vector, contains the PGLOB promoter and the DNA sequence encoding the signal sequence of the basic 7S globulin fused to the cDNA of the human lysosomal enzyme (e.g., GCB SEQ ID NO 1, α-GalA SEQ ID NO 8 and GAA SEQ ID NO 12) deleted of nucleotides coding for the signal sequence of the native enzyme and of any nucleotide not coding the mature enzyme; i.e., in the case of SEQ ID NO 1 nucleotides 1-57, in the case of SEQ ID NO 8 nucleotides 1-116 and, in the case of SEQ ID NO 12, nucleotides 1-426.

Plants suitable for practicing the present invention are those with seeds exhibiting a high protein content, among which *Leguminosae,* cereals and tobacco are particularly suitable.

In fact, as indicated above, protein content in seeds is of about the 25-35% in *Leguminosae*, of about the 10-15% in cereals and of about the 20% in tobacco.

In the preferred embodiment of the present invention tobacco plant was used.

The transformation of the plant cells from which the plant according to the present invention is regenerated may be carried out by any one technique known to a person skilled in the art, like *Agrobacterium-mediated* transformation of leaf discs or of other plant tissues, microinjection of DNA directly into plant cells, electroporation of DNA into plant cell protoplasts, liposome or spheroplast fusion, microprojectile bombardment and the transfection of plant cells or tissues with appropriately engineered plant viruses.

The invention can be practiced by transforming or transfecting plant cells with recombinant expression vectors containing the expression cassette according to the present invention and selecting the transformants or transfectants expressing the enzyme. The transformants may be selected by selection markers commonly known in the state of the art. Plant transformants are selected and induced to regenerate fertile whole plants able to form seeds expressing the lysosomal gene in enzymatically active form following agritechnical methods known in the specific field. In the preferred embodiment of the present invention, the transformation of the plant cells from which the plant is regenerated is carried out with *Agrobacterium* cells made competent by electroporation. The strain with the vector is used to transform leaf discs (LD). Formation first of shoots and then of roots was induced from calluses formed on LD in the presence of selective antibiotic. The plant genetically transformed according to the present invention is a stable transgenic plant whose genetic information, inserted subsequently to the tranforming, is present and expressed (without gene silencing phenomena) in the subsequent generations.

The method for extracting the enzyme produced in seed according to the present invention may be any one standard method for extracting protein from plant tissues known to a person skilled in the art. Said method provides seed grinding in liquid nitrogen in a suitable buffer, centrifuging, supernatant recovering, filtering and further enzyme purification by normal or affinity chromatography.

In a preferred embodiment, the extraction buffer used consists of: sucrose, ascorbic acid, Cys-HCl, Tris-HCl, pH6 EDTA; centrifuging takes place at 2-10°C, preferably at 4°C, at 14.000 rpm for 5-60 min, preferably for 30 min, filter porosity is of about 0.2 µm and the HPLC chromatography column is any column at a weak cationic exchange suitable for the purpose. Preferably, lane Resource Q column (Pharmacia), at a weak cationic exchange with elution in phosphate buffer pH 6 and NaCl gradient 20-100%, is used. For the affinity column, the specific antibodies are bound to affigel-type resins (Biorad) and the elution is carried out with ethylene glycol.

The enzyme extracted from the seed may be used for the preparation of medicaments suitable for enzyme replacement therapy. Therefore, preferably said enzyme will exhibit a high concentration in seed, i.e. between the 0.8 and the 1.5%, preferably between the 0.8 and the 1%.

Optionally, since the in-plant produced enzyme could contain N-linked glycans different from those present in a human product, said enzyme could be modified post-extraction in order to avoid any immunogenic reactions in a patient requiring a regular infusion of the glycoprotein. *In vitro* modifications could remove the xylose residue normally bound to the mannose of plant-produced glycans, according to techniques already used on proteins produced with a CHO cell-based system.

The following examples are meant to provide a more detailed description of the invention without however limiting to them the object that is being claimed.

### EXAMPLES

### Example 1:

### Construction of the vector for the expression of human glucocerebrosidase in plant seeds.

The GCB gene coding for human glucocerebrosidase was cloned, by RT-PCR technique, from mRNA purified from placenta with primers having SEQ ID NO 3 and 4. The gene was isolated, amplifying the DNA having SEQ ID NO 1 with the primers having SEQ ID NO 4 and 5, in its structural portion deleted of the signal peptide (i.e., deleted of the signal sequence of the human gene, namely the nucleotides 1-57 of SEQ ID NO 1) and of the poly-A site (which is not amplified by said primers) and cloned in pGEM®-T (Promega) to form the plasmid named pGEM-GCB (Figure 1). The primers designed for amplification add the SmaI restriction site at 5' and the SacI restriction site at 3'. The sequence of the natural gene obtained, which at sequence control tested identical to the published one (Sorge et al., 1985, Proc. Natl. Acad. Sci. 82: 7289-7293), was cloned in the vector named pPLT2100, in the *Sma*I and *Sac*I sites, under control of the PGLOB promoter (Figure 2) to form the plasmid named pPLT4000 (Figure 3). After accurate control by restriction, the resulting pPLT4000 plasmid was used for genetic transformation of plants.

### Example 2:

### Genetic transformation of plants with pPLT4000 plasmid and general results

pPLT4000 plasmid was transferred in A. *tumefaciens* strain EHA105 cells, made competent, by electroporation. The strain with the plasmid was used to transform about 300 leaf discs of tobacco cultivar (cv) Xanthi. From calluses generated onto leaf discs in the presence of kanamycin first shoot and then root initiation was induced. Rooted plantlets were potted, and at least 150 kanamycin-resistant plants were analysed.

Plants To were tested by PCR (Figure 6) and plants T₁ by Northern (Figure 7) and Western (Figure 8) analyses.

All plants with GCB gene under control of PGLOB promoter led to accumulation of a protein, recognised by GCB-specific antibodies, having a molecular weight equal to 58 kDa corresponding to the glycosilated human protein (Figure 8). The presence of the recombinant protein exclusively in the seed and not in the leaves was assayed in all the examined transgenic plants.

Recombinant GCB protein isolated from seed and purified with HPLC techniques showed to be identical to the natural protein with concern to the molecular weight. Treatment with deglycosilation enzymes confirms the presence of posttranslational modifications in all alike, at present at least in quantitative terms, those present in native glucocerebrosidase (Figure 10).

### EXAMPLE 3:

### Agrobacterium Tumefaciens-mediated tobacco transformation

Day 1: a small amount of *Agrobacterium tumefaciens* of strain EHA 105, taken from a Petri plate culture with a sterile loop so as not to exceed in the amount, thereby avoiding subsequent problems in controlling bacterial proliferation on plated leaf discs, was inoculated in 2 ml of sterile LB. Then, from a healthy tobacco plant cv Xanthi a leaf showing no alteration whatsoever, conversely exhibiting optimal turgor conditions, was taken. The leaf was briefly rinsed with bidistilled water to remove surface impurities, immersed for 8 min in a 20% sodium hypochlorite and 0.1% SDS solution and left to dry under a vertical flow hood. From then on, all steps were carried out under hood. In particular, the leaf was immersed in 95% ethanol and shaken in order to drench its two pages (letting the petiole emerge) for 30-40 sec. The leaf was then allowed to dry out completely.

Discs were obtained from the entire leaf surface with an ethanol-sterilised punch, let fall on plates with antibiotic-free MS10; in particular, the ratio of 30 discs per plate was not exceeded. Next, 2 ml LB + (just inoculated) *Agrobacterium* were poured on plate, and the bacterial suspension was evenly spread over the entire plate with a gentle rotary motion, in order to obtain an homogeneous bacterial distribution among the discs. LB in excess was carefully aspirated with a pipette. At all times in the course of those steps a parallel negative control was performed by means of a plate to which nothing, or only LB was added.

Then plates were incubated at 28°C for 24-48 hours under constant lighting conditions, and bacterial growth was indicated by the appearance of a thin opaque layer spreading over the entire plate.

### Day 2

Leaf discs (LD) were carefully transferred on a plate with MS10 + 500 mg/l cephotaxime, and incubated at 28°C for 6 days under constant lighting conditions. This step determines *Agrobacterium* inactivation.

### Day 8

LD were then carefully transferred on a plate with MS10 + 500 mg/l cephotaxime and 200 mg/l kanamycin, and incubated at 28°C for 14 days, under constant lighting conditions. This step determined a selection of the transformed plants: in fact, kanamycin resistance gene was carried by the plasmid inserted into *Agrobacterium.*

### Day 22

LD, that in the meantime had grown developing a callus, were carefully transferred on a plate with MS10 + 500 mg/l cephotaxime 500 mg/1, 200 mg/l kanamycin and 500 mg/l carbenicillin, and incubated for 6 days. This step determines elimination of the *Agrobacteria* possibly survived to previous antibiotic treatments (a very frequent occurrence).

### Day 28

LD were transferred again on MS10+500 mg/l cephotaxime and 200 mg/l kanamycin, and incubated until shoot appearance. Then, shoots exhibiting at least two leaves were separated from the callous mass and transferred in the radication medium: MSO + 500 mg/l cephotaxime and 200 mg/l kanamycin.

At root appearance, the seedlings were extracted from the plate, freed from agar residues, gently rinsed with running water and planted out in loam and sand (2:1) inside small plastic pots. Soil was previously saturated with water and then pots were covered with transparent plastic lids to preserve high humidity conditions, and placed in a growth chamber at 25°C, with a daily 16-hour lighting period.

The presence of the construct containing the DNA coding for the GCB was assayed by PCR in plants To (Figure 6) and, subsequently, in plants T₁-T₅. The presence of the transcript was assayed by Northern blotting in plants T₁ (Figure 7) as well as the presence of the protein by Western blotting (Figure 8) and, subsequently, in plants T₂ to T₆. The presence of the construct in generations 0-5 and of the transcript as well as of the protein in generations 1-6 demonstrates the stability of the transformation effected, and the absence of gene silencing phenomena in all examined generations subsequent to the first one.

### EXAMPLE 4:

### Purification of glucocerebrosidase protein from different tissues of the plant and assessment of molecular weight.

Extraction of all the tobacco seed proteins was performed grinding the seeds in liquid nitrogen in the presence of an extraction buffer (0.5 M sucrose, 0.1% ascorbic acid, 0.1% Cys-HCl, 0.01 M Tris-HCl, 0.05M EDTA pH 6).

Then the resulting solution was centrifuged at 14.000 rpm for 30 min at 4°C and the supernatant with the soluble proteins was recovered.

The solution was filtered with filters of 0.2 µm porosity, and the glucocerebrosidase partially purified by removing proteins of a <30 KDa molecular weight by centrifugation in Centricon 30 column (Amicon).

The glucocerebrosidase was further purified by HPLC chromatography on Resource Q column (Pharmacia) at a weak cationic exchange, with elution in phosphate buffer pH 6 and NaCl gradient 20-100%. The peak corresponding to glucocerebrosidase eluted at 0.6 M NaCl.

The elution fractions were reunited and filtered in Centricon 30 to remove salt.

For the glucocerebrosidase extraction from tobacco seeds, up to the centrifugation step the Inventors proceeded as in the case of extraction from seed, then the supernatant was additioned with 60% (NH₄)₂SO₄ and left shaking in ice for 60 min.

Then the solution was centrifuged at 14.000 rpm for 15 min at 4°C, the pellet recovered and then resuspended in phosphate buffer pH 6.8.

For the assessment of molecular weight in SDS-PAGE, the staining agent (SDS loading buffer) was additioned to the glucocerebrosidase sample (20µl) and the samples were loaded onto 10% polyacrylamide minigels. Running conditions were: initially 10mA, and 20mA for the entire run, in Tris-glycine 1x buffer. Then the gel was stained by Silver staining technique and the molecular weight assessed referring to molecular weight standards.

### EXAMPLE 5:

### Western analysis of the glucocerebrosidase protein produced in plant and deglycosilation thereof.

Glucocerebrosidase purified from seed according to example 5, after electrophoretic separation on acrylamide gel, was transferred by electroblotting (buffer 25mM Tris, 192 mM glycine, 20% methanol, 45 V at 4°C) onto a nitrocellulose membrane (BA85 Schleicher and Schull).

The membrane with the immobilised protein was shaken for 60 min in TBS-T 5% Skim milk solution and then, after at least three rinsings with TBS-T, the membrane was incubated for 60' at room temperature, shaken with TBS-T 5% Skim milk and primary antibody (rabbit polyclonal antibody specific for human GCB) in a 1:2500 ratio.

After reaction with the primary antibody, the membrane was rinsed at least 3 times with TBS-T and then incubated, shaken for 60' with the secondary antibody (Peroxidase-conjugated anti-rabbit IgG), always in TBS-T 5% Skim milk solution, in a 1:12.000 ratio.

After reaction with the secondary antibody, the membrane was rinsed at least 3 times with TBS-T and placed in contact with Amersham's chemiluminescence kit ECL^{™} solutions.

Then the membrane was exposed in contact with a photoplate (Hyperfilm^{™} MP, Amersham) in darkroom for variable lengths of time (Figure 8).

Deglycosilation with N-glycosidase A and F enzymes (Calbiochem, Boehringer Mannheim) was carried out using 10 µl b/v of glycopeptide (10 µg) denatured in 0.1% SDS brought to boiling point for 2 min.

90 µl of buffer (20 mM phosphate buffer pH 7.2, 50 mM EDTA pH 8, 10 mM sodium azide, 0.5% NP40, 1% β-mercaptoethanol) were additioned to this solution, which was brought to boiling point again for 2 min, then cooled at 37°C. To the resulting 100 µl, 1 U of N glycosidase F and 1 U of N glycosilase A were additioned, and let incubate at 37°C for 18 hours. Then the reaction product was analysed on SDS-PAGE gel and the glucocerebrosidase protein detected by Western technique (Figure 10).

### Example 6:

### Assessment of enzymatic activity of the protein produced and accumulated in seed.

The enzymatic assay was carried out in 200 µl of an 100 mM buffer K phosphate, pH 5, 0.15% triton x-100, 0.125% Na taurocolate, 4-MUG (4 methyl-umbelliferyl glucopyranoside) solution, at 37°C for 60'.

The reaction was terminated by addition of 1 ml of a 0.1M glycine solution pH 10 and the reading was carried out at the spectrophotometer with 340 and 448 nm wavelengths. The calibration curve of the instrument was effected using a commercial enzyme product. For each sample, the assay was repeated in triplicate using 250 ng of partially purified protein. The results are reported in Table 1.

**Table 1. β-glucocerebrosidase enzymatic activity measured in total extracts of proteins from tobacco seed.**

| Tobacco line | Fluorescence |
|---|---|
| WT | 1.4 ± 0.6 |
| SG1 | 26.1 ± 1.5 |
| SG2 | 34.7 ± 1.8 |
| SG10 | 37.0 ± 1.5 |
| SG3 | 21.4 ± 0.9 |
| SG4 | 22.5 ± 1.1 |
| SG17 | 43.9 ± 1.7 |
| SG20 | 18.8 ± 1.2 |
| SG22 | 21.8 ± 1.4 |
| SG31 | 28.9 ± 1.6 |
| SGB | 19.2 ± 1.0 |

### Example 7:

### Quantitative determination of the protein

The assessment of human GCB accumulation capability of the various transgenic lines was carried out comparing the amount of GCB protein in seed to known amounts of commercial GCB. Protein amount was determined after electrophoretic separation on SDS-PAGE gel and detection with a polyclonal primary antibody, produced in rabbit and specific for human GCB and with a peroxidase-conjugated anti-rabbit IgG secondary antibody. The specific band was detected with a scanner, and the protein amount was determined comparing the intensity of the band to that of bands containing a known amount of protein purified and present in the same gel at different dilutions. Thus, tobacco lines were identified whose protein extract produces 8-10 µg GCB per mg of seed-extracted total proteins, i.e. corresponding to an amount ranging from the 0.8% to the 1% of the total proteins of the seed.

### Example 8:

### Construction of the vector for the expression of human α-galactosidase A in plant seeds.

The GLA gene coding for human α-galactosidase A was cloned, by RT-PCR technique, from mRNA purified from placenta, with primers having SEQ ID NO 10 and 11. The gene was isolated, in its structural portion also deleted of the signal peptide (i.e. deleted of nucleotides 1-116 of SEQ ID NO 8) and of the poly-A site (not amplified by said primers) by amplifying the DNA having SEQ ID NO 8 starting from nucleotide 117 with the same primers, and cloned in pGEM®-T (Promega) to form the plasmid named pGEM-GLA. The primers designed for the amplification add the *BamH*I restriction site at 5' and the *EcoRV* restriction site at 3'. The natural gene obtained, which at sequence control tested identical to the published one (Tsuji S. et al., 1987, Eur. J. Biochem., 165(2), 275-280) in the portion encoding the mature peptide, was cloned in a vector analogous to the one named pPLT2100 of example 1 under control of the PGLOB promoter to form the plasmid named pPLT4100 (Figure 4). After accurate control by restriction, the resulting pPLT4100 plasmid was used for genetic transformation of plants.

### Example 9:

### Genetic transformation of plants with pPLT4100 plasmid

Tobacco plant transformation was carried out using the vector named pPLT4100 according to the same methodologies used in examples 2 and 3.

The presence of the construct containing the DNA coding for GLA was assayed by PCR in plants To (Figure 11).

### Example 10:

### Construction of the vector for the expression of human acid α-glucosidase in plant seeds.

The GAA gene coding for human acid □-glucosidase was cloned deleted of the region encoding the signal peptide, by RT-PCR technique, from mRNA purified from placenta, with the primers having sequences SEQ ID NO 14 and 15. The gene, in its structural portion deleted of the signal peptide as well (i.e. deleted of nucleotides 1-426 of SEQ ID NO 12) and of the poly-A site (not amplified by said primers) was isolated, amplifying the DNA having SEQ ID NO 12 starting from nucleotide 427 with the same primers, and cloned in pGEM®-T (Promega) to form the plasmid named pGEM-GLA. The primers designed for the amplification add the restriction site *EcoRV* to 5' and to 3'. The natural gene obtained, which at sequence control tested identical to the published one (Hoefsloot L. H. et al., 1988, EMBO JOURNAL 7(6), 1697-1704) in the portion encoding the mature peptide, was cloned in a vector analogous to that named pPLT2100 of example 1 under control of the PGLOB promoter to form the plasmid named pPLT4200 (Figure 5). After accurate control by restriction, the resulting pPLT4200 plasmid was used for genetic transformation of plants.

### Comparative example:

### Absence of GCB expression in seed using the 35S promoter of the Cauliflower mosaic virus

In order to assess operation of the 35S promoter of the Cauliflower mosaic virus indicated in U.S. Pat. 5,929,304 as promoter suitable for expressing lysosomal enzymes, tobacco plants were transformed using a recombinant expression vector comprising the sequence SEQ ID NO 1 under control of the promoter 35S. The proteins were extracted according to standard methods and the protein product was analysed by Western technique under chemiluminescence using a human GCB-specific rabbit polyclonal antibody as primary antibody, and a peroxidase-conjugated anti-rabbit IgG as secondary antibody. The results, reported in Figure 11, demonstrate that using the 35S promoter for expressing human glucocerebrosidase no accumulation of stable protein in the seed is obtained.

### SEQUENCE LISTING

SEQ ID NO 1:
   cDNA nucleotide sequnce of the GCB gene.
   Underlined sequence: signal peptide 1-57
   Mature peptide: 58-1548 
1          atggc tggcagcctc acaggtttgc ttctacttca ggcagtgtcg tgggcatcag
56         gtgcccgccc ctgcatccct aaaagcttcg gctacagctc ggtggtgtgt
106        gtctgcaatg ccacatactg tgactccttt gaccccccga cctttcctgc
156        ccttggtacc ttcagccgct atgagagtac acgcagtggg cgacggatgg
206        agctgagtat ggggcccatc caggctaatc acacgggcac aggcctgcta
256        ctgaccctgc agccagaaca gaagttccag aaagtgaagg gatttggagg
306        ggccatgaca gatgctgctg ctctcaacat ccttgccctg tcaccccctg
356        cccaaaattt gctacttaaa tcgtacttct ctgaagaagg aatcggatat
406        aacatcatcc gggtacccat ggccagctgt gacttctcca tccgcaccta
456        cacctatgca gacacccctg atgatttcca gttgcacaac ttcagcctcc
506        cagaggaaga taccaagctc aagatacccc tgattcaccg agccctgcag
556        ttggcccagc gtcccgtttc actccttgcc agcccctgga catcacccac
606        ttggctcaag accaatggag cggtgaatgg gaaggggtca ctcaagggac
656        agcccggaga catctaccac cagacctggg ccagatactt tgtgaagttc
706        ctggatgcct atgctgagca caagttacag ttctgggcag tgacagctga
756        aaatgagcct tctgctgggc tgttgagtgg ataccccttc cagtgcctgg
806        gcttcacccc tgaacatcag cgagacttca ttgcccgtga cctaggtcct
856        accctcgcca acagtactca ccacaatgtc cgcctactca tgctggatga
906        ccaacgcttg ctgctgcccc actgggcaaa ggtggtactg acagacccag
956        aagcagctaa atatgttcat ggcattgctg tacattggta cctggacttt
1006       ctggctccag ccaaagccac cctaggggag acacaccgcc tgttccccaa
1056       caccatgctc tttgcctcag aggcctgtgt gggctccaag ttctgggagc
1106       agagtgtgcg gctaggctcc tgggatcgag ggatgcagta cagccacagc
1156       atcatcacga acctcctgta ccatgtggtc ggctggaccg actggaacct
1206       tgccctgaac cccgaaggag gacccaattg ggtgcgtaac tttgtcgaca
1256       gtcccatcat tgtagacatc accaaggaca cgttttacaa acagcccatg
1306       ttctaccacc ttggccactt cagcaagttc attcctgagg gctcccagag
1356       agtggggctg gttgccagtc agaagaacga cctggacgca gtggcactga
1406       tgcatcccga tggctctgct gttgtggtcg tgctaaaccg ctcctctaag
1456       gatgtgcctc ttaccatcaa ggatcctgct gtgggcttcc tggagacaat
1506       ctcacctggc tactccattc acacctacct gtggcatcgc cagtga
SEQ ID NO 2: Amino acid sequence codifing human GCB and native signal peptide. 
Met Ala Gly Ser Leu Thr Gly Leu Leu Leu Leu Gln Ala Val Ser Trp
Ala Ser Gly Ala Arg Pro Cys Ile Pro Lys Ser Phe Gly Tyr Ser Ser
Val Val Cys Val Cys Asn Ala Thr Tyr Cys Asp Ser Phe Asp Pro Pro
Thr Phe Pro Ala Leu Gly Thr Phe Ser Arg Tyr Glu Ser Thr Arg Ser
Gly Arg Arg Met Glu Leu Ser Met Gly Pro Ile Gln Ala Asn His Thr
Gly Thr Gly Leu Leu Leu Thr Leu Gln Pro Glu Gln Lys Phe Gln Lys
Val Lys Gly Phe Gly Gly Ala Met Thr Asp Ala Ala Ala Leu Asn Ile
Leu Ala Leu Ser Pro Pro Ala Gln Asn Leu Leu Leu Lys Ser Tyr Phe
Ser Glu Glu Gly Ile Gly Tyr Asn Ile Ile Arg Val Pro Met Ala Ser
Cys Asp Phe Ser Ile Arg Thr Tyr Thr Tyr Ala Asp Thr Pro Asp Asp
Phe Gln Leu His Asn Phe Ser Leu Pro Glu Glu Asp Thr Lys Leu Lys
Ile Pro Leu Ile His Arg Ala Leu Gln Leu Ala Gln Arg Pro Val Ser
Leu Leu Ala Ser Pro Trp Thr Ser Pro Thr Trp Leu Lys Thr Asn Gly
Ala Val Asn Gly Lys Gly Ser Leu Lys Gly Gln Pro Gly Asp Ile Tyr
His Gln Thr Trp Ala Arg Tyr Phe Val Lys Phe Leu Asp Ala Tyr Ala
Glu His Lys Leu Gln Phe Trp Ala Val Thr Ala Glu Asn Glu Pro Ser
Ala Gly Leu Leu Ser Gly Tyr Pro Phe Gln Cys Leu Gly Phe Thr Pro
Glu His Gln Arg Asp Phe Ile Ala Arg Asp Leu Gly Pro Thr Leu Ala
Asn Ser Thr His His Asn Val Arg Leu Leu Met Leu Asp Asp Gln Arg
Leu Leu Leu Pro His Trp Ala Lys Val Val Leu Thr Asp Pro Glu Ala
Ala Lys Tyr Val His Gly Ile Ala Val His Trp Tyr Leu Asp Phe Leu
Ala Pro Ala Lys Ala Thr Leu Gly Glu Thr His Arg Leu Phe Pro Asn
Thr Met Leu Phe Ala Ser Glu Ala Cys Val Gly Ser Lys Phe Trp Glu
Gln Ser Val Arg Leu Gly Ser Trp Asp Arg Gly Met Gln Tyr Ser His
Ser Ile Ile Thr Asn Leu Leu Tyr His Val Val Gly Trp Thr Asp Trp
Asn Leu Ala Leu Asn Pro Glu Gly Gly Pro Asn Trp Val Arg Asn Phe
Val Asp Ser Pro Ile Ile Val Asp Ile Thr Lys Asp Thr Phe Tyr Lys
Gln Pro Met Phe Tyr His Leu Gly His Phe Ser Lys Phe Ile Pro Glu
Gly Ser Gln Arg Val Gly Leu Val Ala Ser Gln Lys Asn Asp Leu Asp
Ala Val Ala Leu Met His Pro Asp Gly Ser Ala Val Val Val Val Leu
Asn Arg Ser Ser Lys Asp Val Pro Leu Thr Ile Lys Asp Pro Ala Val
Gly Phe Leu Glu Thr Ile Ser Pro Gly Tyr Ser Ile His Thr Tyr Leu
Trp His Arg Gln
SEQ ID NO 3: forward primer for GCB amplification
   5': tctagaatggctggcagcctcacaggt
SEQ ID NO 4: reverse primer for GCB amplification
   5': gtgtggatggacaccgtagcggtcactctcgag
SEQ ID NO 5: forward primer for GCB amplification
   5': cccgggtgcccgcccctgcatccctaaaagc
SEQ ID NO 6: PGLOB promoter 
1   taaaataatc tatacattaa aaaatttgat tttaaaattt tagaaattca tgattttatt
61  tttttttacc agaaatccgt taatattgtt aaaatattac caactaattt ataaatttta
121 ttttaaggca attaagcatg tttgataaaa tatatatatt gttataaata cttttcaaaa
181 gtataaagtt gatgatggcg tggtggtaga ttattttagt tctaggttcg aatgcaagtt
241 ggtttagaca tttagcctta ttcttttttc taaccaaaat aaatgtaaat ggaaaacctt
301 taggaaaaaa aagaaatcaa aattgaaaac atcatccggt ggagtcgaga agcccacacc
361 cacgtgaccc aacaatatta aaataagagt ttgctctaca gtaaatgcga tactttttta
421 ttcaatactt tttccacttc taaaatcttg gagatttgca ccgttaacta attaagtgtt
481 atatccaacg gtcctaaaaa aacttgtgta ccgtgcctca catttcaact ttgcgcaccc
541 tagaagccgt ctatgtttag gttagtgttt gcaacagttg aagcgcatca ctcaggaggc
601 tacttggtct tgcttttgcg tcttttgttc aatttttcac gtgattttgt tggtgaacac
661 gcgtacttga aacttattat aaattacata attttataag tttcacttct tatataatac
721 ttcattcatg catttataat tttgatgaat aataaagagt ttgttaaaaa atatattatt
781 tcatataata tatagggttt agaatgccaa tttttaaaaa aagaataaaa aaataaatag
841 aataaaatcg aaaaaatgaa atgtaaaaaa tttgaggggg acaaataaaa tatgaaagtc
901 tattatttaa attttccatt agaattctat tttccttagt taatatgagc tagccagttg
961 ggagatacac gaaaatgtca tgaaacagtt gcatgtaggg aaattaatgt agtagaggga
1021 tagcaagaca aaaatccaag ccaagctagc tgctcacgcg aactcgatcc acacgtcctt
1081 tacagagttt caaacggatg aaatctgcat ggcatgcaac taaagcattg ttctcagctg
1141 ccaagtaccc ctcacactca ccaacccttt gtttttctcc ccattgcatg ttaactcaag
1201 tttatccttt ctttgcttct ggaaatttca caagcctcaa acacgtcgac gtccaatctt
1261 gtgaccaaca cggccaaaag aaaagagaat ctcatcccgt tcacacttag ccacttaaag
1321 ctagccaaac ggtgatcttt ctctatatat tgtagctctc taacacaacc aacactacca
1381 ttattcaata ttcaaacctt gctctatact acacacacta gaagaata
SEQ ID NO 7: Soy basic glubulin 7S signal sequence 1 atggcttctat cctccactac tttttagccc tctctctttc ttgctctttt cttttcttct 61 tatccgactc a
SEQ ID NO 8: cDNA nucleotide sequence of the GLA gene Underlined sequence: signal peptide 21-116
   Sequece coding for the mature peptide 117-1310 
1 aatgctgtcc ggtcaccgtg acaatgcagc tgaggaaccc agaactacat ctgggctgcg
61 cgcttgcgct tcgcttcctg gccctcgttt cctgggacat ccctggggct agagcactgg
121 acaatggatt ggcaaggacg cctaccatgg gctggctgca ctgggagcgc ttcatgtgca
181 accttgactg ccaggaagag ccagattcct gcatcagtga gaagctcttc atggagatgg
241 cagagctcat ggtctcagaa ggctggaagg atgcaggtta tgagtacctc tgcattgatg
301 actgttggat ggctccccaa agagattcag aaggcagact tcaggcagac cctcagcgct
361 ttcctcatgg gattcgccag ctagctaatt atgttcacag caaaggactg aagctaggga
421 tttatgcaga tgttggaaat aaaacctgcg caggcttccc tgggagtttt ggatactacg
481 acattgatgc ccagaccttt gctgactggg gagtagatct gctaaaattt gatggttgtt
541 actgtgacag tttggaaaat ttggcagatg gttataagca catgtccttg gccctgaata
601 ggactggcag aagcattgtg tactcctgtg agtggcctct ttatatgtgg ccctttcaaa
661 agcccaatta tacagaaatc cgacagtact gcaatcactg gcgaaatttt gctgacattg
721 atgattcctg gaaaagtata aagagtatct tggactggac atcttttaac caggagagaa
781 ttgttgatgt tgctggacca gggggttgga atgacccaga tatgttagtg attggcaact
841 ttggcctcag ctggaatcag caagtaactc agatggccct ctgggctatc atggctgctc
901 ctttattcat gtctaatgac ctccgacaca tcagccctca agccaaagct ctccttcagg
961 ataaggacgt aattgccatc aatcaggacc ccttgggcaa gcaagggtac cagcttagac
1021 agggagacaa ctttgaagtg tgggaacgac ctctctcagg cttagcctgg gctgtagcta
1081 tgataaaccg gcaggagatt ggtggacctc gctcttatac catcgcagtt gcttccctgg
1141 gtaaaggagt ggcctgtaat cctgcctgct tcatcacaca gctcctccct gtgaaaagga
1201 agctagggtt ctatgaatgg acttcaaggt taagaagtca cataaatccc acaggcactg
1261 ttttgcttca gctagaaaat acaatgcaga tgtcattaaa agacttactt taaaatgtt
SEQ ID NO 9: Amino acid sequence coding for GLA and native signal peptide 
Thr Met Gln Leu Arg Asn Pro Glu Leu His Leu Gly Cys Ala Leu Ala
Leu Arg Phe Leu Ala Leu Val Ser Trp Asp Ile Pro Gly Ala Arg Ala
Leu Asp Asn Gly Leu Ala Arg Thr Pro Thr Met Gly Trp Leu His Trp
Glu Arg Phe Met Cys Asn Leu Asp Cys Gln Glu Glu Pro Asp Ser Cys
 
Ile Ser Glu Lys Leu Phe Met Glu Met Ala Glu Leu Met Val Ser Glu
Gly Trp Lys Asp Ala Gly Tyr Glu Tyr Leu Cys Ile Asp Asp Cys Trp
Met Ala Pro Gln Arg Asp Ser Glu Gly Arg Leu Gln Ala Asp Pro Gln
Arg Phe Pro His Gly Ile Arg Gln Leu Ala Asn Tyr Val His Ser Lys
Gly Leu Lys Leu Gly Ile Tyr Ala Asp Val Gly Asn Lys Thr Cys Ala
Gly Phe Pro Gly Ser Phe Gly Tyr Tyr Asp Ile Asp Ala Gln Thr Phe
Ala Asp Trp Gly Val Asp Leu Leu Lys Phe Asp Gly Cys Tyr Cys Asp
Ser Leu Glu Asn Leu Ala Asp Gly Tyr Lys His Met Ser Leu Ala Leu
Asn Arg Thr Gly Arg Ser Ile Val Tyr Ser Cys Glu Trp Pro Leu Tyr
Met Trp Pro Phe Gln Lys Pro Asn Tyr Thr Glu Ile Arg Gln Tyr Cys
Asn His Trp Arg Asn Phe Ala Asp Ile Asp Asp Ser Trp Lys Ser Ile
Lys Ser Ile Leu Asp Trp Thr Ser Phe Asn Gln Glu Arg Ile Val Asp
Val Ala Gly Pro Gly Gly Trp Asn Asp Pro Asp Met Leu Val Ile Gly
Asn Phe Gly Leu Ser Trp Asn Gln Gln Val Thr Gln Met Ala Leu Trp
Ala Ile Met Ala Ala Pro Leu Phe Met Ser Asn Asp Leu Arg His Ile
Ser Pro Gln Ala Lys Ala Leu Leu Gln Asp Lys Asp Val Ile Ala Ile
Asn Gln Asp Pro Leu Gly Lys Gln Gly Tyr Gln Leu Arg Gln Gly Asp
Asn Phe Glu Val Trp Glu Arg Pro Leu Ser Gly Leu Ala Trp Ala Val
Ala Met Ile Asn Arg Gln Glu Ile Gly Gly Pro Arg Ser Tyr Thr Ile
Ala Val Ala Ser Leu Gly Lys Gly Val Ala Cys Asn Pro Ala Cys Phe
Ile Thr Gln Leu Leu Pro Val Lys Arg Lys Leu Gly Phe Tyr Glu Trp
Thr Ser Arg Leu Arg Ser His Ile Asn Pro Thr Gly Thr Val Leu Leu
Gln Leu Glu Asn Thr Met Gln Met Ser Leu Lys Asp Leu Leu
SEQ ID NO 10: Forward primer for GLA amplification 5': ggatccctggacaatggattggcaaggac
SEQ ID NO 11: Reverse primer for GLA amplification
   5': gtctacagtaattttctgaatgaaattctatag
SEQ ID NO 12: cDNA GAA.
   Underlined sequence: signal peptide 220-426
   Sequence coding for the mature peptide 427-3075 
1 cagttgggaa agctgaggtt gtcgccgggg ccgcgggtgg aggtcgggga tgaggcagca

61 ggtaggacag tgacctcggt gacgcgaagg accccggcca cctctaggtt ctcctcgtcc
 
121 gcccgttgtt cagcgaggga ggctctgggc ctgccgcagc tgacggggaa actgaggcac
181 ggagcgggcc tgtaggagct gtccaggcca tctccaacca tgggagtgag gcacccgccc
241 tgctcccacc ggctcctggc cgtctgcgcc ctcgtgtcct tggcaaccgc tgcactcctg
301 gggcacatcc tactccatga tttcctgctg gttccccgag agctgagtgg ctcctcccca
361 gtcctggagg agactcaccc agctcaccag cagggagcca gcagaccagg gccccgggat
421 gcccaggcac accccggccg tcccagagca gtgcccacac agtgcgacgt cccccccaac
481 agccgcttcg attgcgcccc tgacaaggcc atcacccagg aacagtgcga ggcccgcggc
541 tgctgctaca tccctgcaaa gcaggggctg cagggagccc agatggggca gccctggtgc
601 ttcttcccac ccagctaccc cagctacaag ctggagaacc tgagctcctc tgaaatgggc
661 tacacggcca ccctgacccg taccaccccc accttcttcc ccaaggacat cctgaccctg
721 cggctggacg tgatgatgga gactgagaac cgcctccact tcacgatcaa agatccagct
781 aacaggcgct acgaggtgcc cttggagacc ccgcgtgtcc acagccgggc accgtcccca
841 ctctacagcg tggagttctc cgaggagccc ttcggggtga tcgtgcaccg gcagctggac
901 ggccgcgtgc tgctgaacac gacggtggcg cccctgttct ttgcggacca gttccttcag
961 ctgtccacct cgctgccctc gcagtatatc acaggcctcg ccgagcacct cagtcccctg
1021 atgctcagca ccagctggac caggatcacc ctgtggaacc gggaccttgc gcccacgccc
1081 ggtgcgaacc tctacgggtc tcaccctttc tacctggcgc tggaggacgg cgggtcggca
1141 cacggggtgt tcctgctaaa cagcaatgcc atggatgtgg tcctgcagcc gagccctgcc
1201 cttagctgga ggtcgacagg tgggatcctg gatgtctaca tcttcctggg cccagagccc
1261 aagagcgtgg tgcagcagta cctggacgtt gtgggatacc cgttcatgcc gccatactgg
1321 ggcctgggct tccacctgtg ccgctggggc tactcctcca ccgctatcac ccgccaggtg
1381 gtggagaaca tgaccagggc ccacttcccc ctggacgtcc aatggaacga cctggactac
1441 atggactccc ggagggactt cacgttcaac aaggatggct tccgggactt cccggccatg
1501 gtgcaggagc tgcaccaggg cggccggcgc tacatgatga tcgtggatcc tgccatcagc
1561 agctcgggcc ctgccgggag ctacaggccc tacgacgagg gtctgcggag gggggtttc
1621 atcaccaacg agaccggcca gccgctgatt gggaaggtat ggcccgggtc cactgccttc
1681 cccgacttca ccaaccccac agccctggcc tggtgggagg acatggtggc tgagttccat
1741 gaccaggtgc ccttcgacgg catgtggatt gacatgaacg agccttccaa cttcatcaga
1801 ggctctgagg acggctgccc caacaatgag ctggagaacc caccctacgt gcctggggtg
1861 gttgggggga ccctccaggc ggccaccatc tgtgcctcca gccaccagtt tctctccaca
1921 cactacaacc tgcacaacct ctacggcctg accgaagcca tcgcctccca cagggcgctg
1981 gtgaaggctc gggggacacg cccatttgtg atctcccgct cgacctttgc tggccacggc
2041 cgatacgccg gccactggac gggggacgtg tggagctcct gggagcagct cgcctcctcc
2101 gtgccagaaa tcctgcagtt taacctgctg ggggtgcctc tggtcggggc cgacgtctgc
2161 ggcttcctgg gcaacacctc agaggagctg tgtgtgcgct ggacccagct gggggccttc
2221 taccccttca tgcggaacca caacagcctg ctcagtctgc cccaggagcc gtacagcttc
2281 agcgagccgg cccagcaggc catgaggaag gccctcaccc tgcgctacgc actcctcccc
 
2341 cacctctaca cactgttcca ccaggcccac gtcgcggggg agaccgtggc ccggcccctc
2401 ttcctggagt tccccaagga ctctagcacc tggactgtgg accaccagct cctgtggggg
2461 gaggccctgc tcatcacccc agtgctccag gccgggaagg ccgaagtgac tggctacttc
2521 cccttgggca catggtacga cctgcagacg gtgccaatag aggcccttgg cagcctccca
2581 cccccacctg cagctccccg tgagccagcc atccacagcg aggggcagtg ggtgacgctg
2641 ccggcccccc tggacaccat caacgtccac ctccgggctg ggtacatcat ccccctgcag
2701 ggccctggcc tcacaaccac agagtcccgc cagcagccca tggccctggc tgtggccctg
2761 accaagggtg gagaggcccg aggggagctg ttctgggacg atggagagag cctggaagtg
2821 ctggagcgag gggcctacac acaggtcatc ttcctggcca ggaataacac gatcgtgaat
2881 gagctggtac gtgtgaccag tgagggagct ggcctgcagc tgcagaaggt gactgtcctg
2941 ggcgtggcca cggcgcccca gcaggtcctc tccaacggtg tccctgtctc caacttcacc
3001 tacagccccg acaccaaggt cctggacatc tgtgtctcgc tgttgatggg agagcagttt
3061 ctcgtcagct ggtgttagcc gggcggagtg tgttagtctc tccagaggga ggctggttcc
3121 ccagggaagc agagcctgtg tgcgggcagc agctgtgtgc gggcctgggg gttgcatgtg
3181 tcacctggag ctgggcacta accattccaa gccgccgcat cgcttgtttc cacctcctgg
3241 gccggggctc tggcccccaa cgtgtctagg agagctttct ccctagatcg cactgtgggc
3301 cggggcctgg agggctgctc tgtgttaata agattgtaag gtttgccctc ctcacctgtt
3361 gccggcatgc gggtagtatt agccaccccc ctccatctgt tcccagcacc ggagaagggg
3421 gtgctcaggt ggaggtgtgg ggtatgcacc tgagctcctg cttcgcgcct gctgctctgc
3481 cccaacgcga ccgcttcccg gctgcccaga gggctggatg cctgccggtc cccgagcaag
3541 cctgggaact caggaaaatt cacaggactt gggagattct aaatcttaag tgcaattatt
3601 ttaataaaag gggcatttgg aatc
SEQ ID NO 13: Amino acid sequence of human GAA and native signal peptide 
Ala His Pro Gly Arg Pro Arg Ala Val Pro Thr Gln Cys Asp Val Pro
Pro Asn Ser Arg Phe Asp Cys Ala Pro Asp Lys Ala Ile Thr Gln Glu
Gln Cys Glu Ala Arg Gly Cys Cys Tyr Ile Pro Ala Lys Gln Gly Leu
Gln Gly Ala Gln Met Gly Gln Pro Trp Cys Phe Phe Pro Pro Ser Tyr
Pro Ser Tyr Lys Leu Glu Asn Leu Ser Ser Ser Glu Met Gly Tyr Thr
Ala Thr Leu Thr Arg Thr Thr Pro Thr Phe Phe Pro Lys Asp Ile Leu
Thr Leu Arg Leu Asp Val Met Met Glu Thr Glu Asn Arg Leu His Phe
Thr Ile Lys Asp Pro Ala Asn Arg Arg Tyr Glu Val Pro Leu Glu Thr
Pro Arg Val His Ser Arg Ala Pro Ser Pro Leu Tyr Ser Val Glu Phe
Ser Glu Glu Pro Phe Gly Val Ile Val His Arg Gln Leu Asp Gly Arg
Val Leu Leu Asn Thr Thr Val Ala Pro Leu Phe Phe Ala Asp Gln Phe
Leu Gln Leu Ser Thr Ser Leu Pro Ser Gln Tyr Ile Thr Gly Leu Ala
 
Glu His Leu Ser Pro Leu Met Leu Ser Thr Ser Trp Thr Arg Ile Thr
Leu Trp Asn Arg Asp Leu Ala Pro Thr Pro Gly Ala Asn Leu Tyr Gly
Ser His Pro Phe Tyr Leu Ala Leu Glu Asp Gly Gly Ser Ala His Gly
Val Phe Leu Leu Asn Ser Asn Ala Met Asp Val Val Leu Gln Pro Ser
Pro Ala Leu Ser Trp Arg Ser Thr Gly Gly Ile Leu Asp Val Tyr Ile
Phe Leu Gly Pro Glu Pro Lys Ser Val Val Gln Gln Tyr Leu Asp Val
Val Gly Tyr Pro Phe Met Pro Pro Tyr Trp Gly Leu Gly Phe His Leu
Cys Arg Trp Gly Tyr Ser Ser Thr Ala Ile Thr Arg Gln Val Val Glu
Asn Met Thr Arg Ala His Phe Pro Leu Asp Val Gln Trp Asn Asp Leu
Asp Tyr Met Asp Ser Arg Arg Asp Phe Thr Phe Asn Lys Asp Gly Phe
Arg Asp Phe Pro Ala Met Val Gln Glu Leu His Gln Gly Gly Arg Arg
Tyr Met Met Ile Val Asp Pro Ala Ile Ser Ser Ser Gly Pro Ala Gly
Ser Tyr Arg Pro Tyr Asp Glu Gly Leu Arg Arg Gly Val Phe Ile Thr
Asn Glu Thr Gly Gln Pro Leu Ile Gly Lys Val Trp Pro Gly Ser Thr
Ala Phe Pro Asp Phe Thr Asn Pro Thr Ala Leu Ala Trp Trp Glu Asp
Met Val Ala Glu Phe His Asp Gln Val Pro Phe Asp Gly Met Trp Ile
Asp Met Asn Glu Pro Ser Asn Phe Ile Arg Gly Ser Glu Asp Gly Cys
Pro Asn Asn Glu Leu Glu Asn Pro Pro Tyr Val Pro Gly Val Val Gly
Gly Thr Leu Gln Ala Ala Thr Ile Cys Ala Ser Ser His Gln Phe Leu
Ser Thr His Tyr Asn Leu His Asn Leu Tyr Gly Leu Thr Glu Ala Ile
Ala Ser His Arg Ala Leu Val Lys Ala Arg Gly Thr Arg Pro Phe Val
Ile Ser Arg Ser Thr Phe Ala Gly His Gly Arg Tyr Ala Gly His Trp
Thr Gly Asp Val Trp Ser Ser Trp Glu Gln Leu Ala Ser Ser Val Pro
Glu Ile Leu Gln Phe Asn Leu Leu Gly Val Pro Leu Val Gly Ala Asp
Val Cys Gly Phe Leu Gly Asn Thr Ser Glu Glu Leu Cys Val Arg Trp
Thr Gln Leu Gly Ala Phe Tyr Pro Phe Met Arg Asn His Asn Ser Leu
Leu Ser Leu Pro Gln Glu Pro Tyr Ser Phe Ser Glu Pro Ala Gln Gln
Ala Met Arg Lys Ala Leu Thr Leu Arg Tyr Ala Leu Leu Pro His Leu
Tyr Thr Leu Phe His Gln Ala His Val Ala Gly Glu Thr Val Ala Arg
Pro Leu Phe Leu Glu Phe Pro Lys Asp Ser Ser Thr Trp Thr Val Asp
His Gln Leu Leu Trp Gly Glu Ala Leu Leu Ile Thr Pro Val Leu Gln
Ala Gly Lys Ala Glu Val Thr Gly Tyr Phe Pro Leu Gly Thr Trp Tyr
Asp Leu Gln Thr Val Pro Ile Glu Ala Leu Gly Ser Leu Pro Pro Pro
Pro Ala Ala Pro Arg Glu Pro Ala Ile His Ser Glu Gly Gln Trp Val
Thr Leu Pro Ala Pro Leu Asp Thr Ile Asn Val His Leu Arg Ala Gly
Tyr Ile Ile Pro Leu Gln Gly Pro Gly Leu Thr Thr Thr Glu Ser Arg
Gln Gln Pro Met Ala Leu Ala Val Ala Leu Thr Lys Gly Gly Glu Ala
 
Arg Gly Glu Leu Phe Trp Asp Asp Gly Glu Ser Leu Glu Val Leu Glu
Arg Gly Ala Tyr Thr Gln Val Ile Phe Leu Ala Arg Asn Asn Thr Ile
Val Asn Glu Leu Val Arg Val Thr Ser Glu Gly Ala Gly Leu Gln Leu
Gln Lys Val Thr Val Leu Gly Val Ala Thr Ala Pro Gln Gln Val Leu
Ser Asn Gly Val Pro Val Ser Asn Phe Thr Tyr Ser Pro Asp Thr Lys
Val Leu Asp Ile Cys Val Ser Leu Leu Met Gly Glu Gln Phe Leu Val
Ser Trp Cys
SEQ ID NO 14: Forward primer for GAA amplification
   5': gatatctgcacaccccggccgtcccag
SEQ ID NO 15: Reverse primer for GAA amplification
   5': gtcaaagagcagtcgaccacaatcctatag

## Claims

1. A genetically transformed plant able to produce a lysosomal enzyme of animal or human origin, **characterised in that**:
- said plant is transformed via the use of an expression vector comprising:
a. a promoter of a plant gene specific for the expression in seed storage organs and stage-specific;
b. a DNA sequence encoding the signal sequence of a plant protein able to dispatch said lysosomal enzyme to seed storage organs and to provide the post-translational modifications required for the expression of the enzyme in active form;
c. a DNA sequence encoding said lysosomal enzyme deleted of the native signal sequence;
- said enzyme is expressed in seed storage tissues in enzymatically active form and in an amount of at least the 0.8% of the total proteins of the seed.

2. The plant according to claim 1, **characterised in that** the expression vector is a plasmid.

3. The plant according to claims 1 or 2, **characterised in that** the promoter derives from the gene of 7S soy globulin.

4. The plant according to any one of the claims 1 to 3, **characterised in that** the DNA sequence encoding the signal sequence derives from the gene of the 7S soy globulin and is fused to the sequence encoding the structural portion of the mature lysosomal enzyme deleted of the native signal sequence.

5. The plant according to any one of the claims 1 to 4, wherein the lysosomal enzyme expressed in enzymatically active form in seed storage tissues is:
α-N-acetylgalactosaminidase, acid lipase, aryl sulfatase A, aspartylglycosaminidase, ceramidase, α-fucosidase, α-galactosidase A, β-galactosidase, galactosylceramidase, glucocerebrosidase, α-glucosidase, β-glucuronidase, heparin N-sulfatase, β-hexosaminidase, iduronate sulfatase, α-L-iduronidase, α-mannosidase, mannosidase, sialidase and sphingomyelinase.

6. The plant according to any one of the claims 1 to 5, wherein said plant is a *Leguminosa,* cereal, or tobacco.

7. A method for producing the genetically transformed plant able to produce a lysosomal enzyme according to any one of the claims 1 to 6, **characterised in that**:
- plant cells are transformed via the use of an expression vector comprising:
a. a promoter of a plant gene specific for the expression in seed storage organs and stage-specific;
b. a DNA sequence encoding the signal sequence of a plant protein able to dispatch said lysosomal enzyme to seed storage organs and to provide the post-translational modifications required for expression of the enzyme in active form;
c. a DNA sequence encoding said lysosomal enzyme deleted of the native signal sequence;
- said cells are used to regenerate said transformed plant.

8. The method according to claim 7, wherein said plant is a *Leguminosa,* cereal, or tobacco.

9. A seed of genetically modified plant able to express a lysosomal enzyme, **characterised in that**:
- said seed contains an expression vector comprising:
a. a promoter of a plant gene specific for the expression in seed storage organs and stage-specific;
b. a DNA sequence encoding the signal sequence of a plant protein able to dispatch said lysosomal enzyme to seed storage organs and to provide the post-translational modifications required for the expression of the enzyme in active form;
c. a DNA sequence encoding said lysosomal enzyme deleted of the native signal sequence;
- said enzyme is contained in seed storage tissues in enzymatically active form and in the amount of at least the 0.8% of the seed total proteins.

10. The seed according to claim 9, **characterised in that** the expression vector is a plasmid.

11. The seed according to claims 9 or 10, **characterised in that** the promoter derives from the gene of 7S soy globulin.

12. The seed according to any one of the claims 9 to 11, **characterised in that** the DNA sequence encoding the signal sequence derives from the gene of 7S soy globulin and is fused to the sequence encoding the structural portion of the mature lysosomal enzyme deleted of the native signal sequence.

13. The seed according to any one of the claims 9 to 12, **characterised in that** the lysosomal enzyme expressed in enzymatically active form in seed storage tissues is:
α-N-acetylgalactosaminidase, acid lipase, aryl sulfatase A, aspartylglycosaminidase, ceramidase, α-fucosidase, α-galactosidase A, β-galactosidase, galactosylceramidase, glucocerebrosidase, α-glucosidase, β-glucuronidase, heparin N-sulfatase, β-hexosaminidase, iduronate sulfatase, α-L-iduronidase, α-mannosidase, β-mannosidase, sialidase and sphingomyelinase.

14. The seed according to any one of the claims 9 to 13, wherein said seed is of a *Leguminosa,* cereal or tobacco.

15. A method for producing the seed according to any one of the claims 9 to 14, **characterised in that**:
- plant cells are transformed via the use of an expression vector comprising:
a. a promoter of a plant gene specific for the expression in seed storage organs and stage-specific;
b. a DNA sequence encoding the signal sequence of a plant protein able to dispatch said lysosomal enzyme to seed storage organs and to provide the post-translational modifications required for expression of the enzyme in active form;
c. a DNA sequence encoding said lysosomal enzyme deleted of the native signal sequence;
- said cells are used to regenerate transformed plants able to produce said seeds.

16. The method according to claim 15, wherein said seed is of *Leguminosa,* cereal or tobacco.

17. A method for extracting and purifying the lysosomal enzyme in active form contained in the seed according to any one of the claims 9 to 14, **characterised in that**:
a. said seed is ground in liquid nitrogen in the presence of an extraction buffer;
b. the resulting solution is centrifuged;
c. the supernatant is recovered and filtered with filters having a porosity suitable to the enzyme dimensions;
d. the partially purified enzyme is further purified by HPLC chromatography.

18. A use of the seed according to claims 9 to 14, for the preparation of medicaments for enzyme replacement therapies.

19. The use of the seed according to claim 18 for the preparation of a medicament for an enzyme replacement therapy in Gaucher disease.

20. The use of the seed according to claim 18 for the preparation of a medicament for an enzyme replacement therapy in Anderson-Fabry disease.

21. The use of the seed according to claim 18 for the preparation of a medicament for an enzyme replacement therapy in Pompe disease.

22. The use of the seed according to any one of the claims 9 to 14 as means for storing and preserving a lysosomal enzyme in enzymatically active form.

## Patentansprüche

1. Gentechnisch transformierte Pflanze, welche in der Lage ist, ein lysosomales Enzym tierischen oder menschlichen Ursprungs zu erzeugen, **dadurch gekennzeichnet, dass**:
- die Pflanze durch die Verwendung eines Expressionsvektors transformiert ist, welcher umfasst:
(a) einen Promotor eines Pflanzengens, welcher spezifisch ist für die Expression in Samenspeicherorganen, und welcher Stadiumsspezifisch ist;
(b) eine DNA-Sequenz, welche die Signalsequenz eines Pflanzenproteins codiert, welches in der Lage ist, den Transport des lysosomalen Enzyms zu den Samenspeicherorganen zu vermitteln und die post-translationalen Modifizierungen bereitzustellen, welche für die Expression des Enzyms in aktiver Form erforderlich sind;
(c) eine DNA-Sequenz, welche das lysosomale Enzym ohne die native Signalsequenz codiert;
- das Enzym in Samenspeichergeweben in enzymatisch aktiver Form und in einer Menge von mindestens 0,8 % des Gesamtproteingehalts des Samens exprimiert wird.

2. Pflanze nach Anspruch 1, **dadurch gekennzeichnet, dass** der Expressionsvektor ein Plasmid ist.

3. Pflanze nach Anspruch 1 oder 2, **dadurch gekennzeichnet, dass** der Promotor vom Soja-7S-Globulin-Gen stammt.

4. Pflanze nach einem der Ansprüche 1 bis 3, **dadurch gekennzeichnet, dass** die DNA-Sequenz, welche die Signalsequenz codiert, vom Soja-7S-Globulin-Gen stammt, und mit der Sequenz fusioniert ist, welche das Strukturprotein des reifen lysosomalen Enzyms ohne native Signalsequenz codiert.

5. Pflanze nach einem der Ansprüche 1 bis 4, wobei das lysosomale Enzym, welches in enzymatisch aktiver Form in Samenspeichergeweben exprimiert wird, ist:
α-N-Acetylgalactosaminidase, saure Lipase, Arylsulfatase A, Aspartylglycosaminidase, Ceramidase, α-Fucosidase, α-Galactosidase A, β-Galactosidase, Galactosylceramidase, Glucocerebrosidase, α-Glucosidase, β-Glucuronidase, Heparin-N-sulfatase, β-Hexosaminidase, Idunoratsulfatase, α-L-Iduronidase, α-Mannosidase, β-Mannosidase, Sialidase und Sphingomyelinase.

6. Pflanze nach einem der Ansprüche 1 bis 5, wobei die Pflanze eine *Leguminose,* Getreide oder Tabak ist.

7. Verfahren zur Herstellung der gentechnisch transformierten Pflanze nach einem der Ansprüche 1 bis 6, welche in der Lage ist, ein lysosomales Enzym zu erzeugen, **dadurch gekennzeichnet, dass**:
- Pflanzenzellen durch die Verwendung eines Expressionsvektors transformiert werden, umfassend:
(a) einen Promotor eines Pflanzengens, welcher spezifisch ist für die Expression in Samenspeicherorganen und welcher stadiumspezifisch ist;
(b) eine DNA-Sequenz, welche die Signalsequenz eines Pflanzenproteins codiert, welches in der Lage ist, den Transport des lysosomalen Enzyms zu den Samenspeicherorganen zu vermitteln und die post-translationalen Modifizierungen bereitzustellen, welche für die Expression des Enzyms in aktiver Form erforderlich sind;
(c) eine DNA-Sequenz, welche das lysosomale Enzym ohne die native Signalsequenz codiert;
- die Zellen verwendet werden, um die transformierte Pflanze zu regenerieren.

8. Verfahren nach Anspruch 7, wobei die Pflanze eine *Leguminose,* Getreide oder Tabak ist.

9. Samen einer gentechnisch modifizierten Pflanze, welcher in der Lage ist, ein lysosomales Enzym zu exprimieren, **dadurch gekennzeichnet dass**:
- der Samen einen Expressionsvektor enthält, umfassend:
(a) einen Promotor eines Pflanzengens, welcher spezifisch ist für die Expression in Samenspeicherorganen und welcher Stadiumsspezifisch ist;
(b) eine DNA-Sequenz, welche die Signalsequenz eines Pflanzenproteins codiert, welches in der Lage ist, den Transport des lysosomalen Enzyms zu den Samenspeicherorganen zu vermitteln und die post-translationalen Modifizierungen bereitzustellen, welche für die Expression des Enzyms in aktiver Form erforderlich sind;
(c) eine DNA-Sequenz, welche das lysosomale Enzym ohne die native Signalsequenz codiert;
- das Enzym in Samenspeichergeweben in enzymatisch aktiver Form und in einer Menge von mindestens 0,8 % des Gesamtproteingehalts des Samens enthalten ist.

10. Samen nach Anspruch 9, **dadurch gekennzeichnet, dass** der Expressionsvektor ein Plasmid ist.

11. Samen nach Anspruch 9 oder 10, **dadurch gekennzeichnet, dass** der Promotor vom Soja-7S-Globulin-Gen stammt.

12. Samen nach einem der Ansprüche 9 bis 11, **dadurch gekennzeichnet, dass** die DNA-Sequenz, welche die Signalsequenz codiert, vom Soja-7S-Globulin-Gen stammt und mit der Sequenz fusioniert ist, welche das Strukturprotein des reifen lysosomalen Enzyms ohne native Signalsequenz codiert.

13. Samen nach einem der Ansprüche 9 bis 12, **dadurch gekennzeichnet, dass** das lysosomale Enzym, welches in enzymatisch aktiver Form in Samenspeichergeweben exprimiert wird, ist:
α-N-Acetylgalactosaminidase, saure Lipase, Arylsulfatase A, Aspartylglycosaminidase, Ceramidase, α-Fucosidase, α-Galactosidase A, β-Galactosidase, Galactosylceramidase, Glucocerebrosidase, α-Glucosidase, β-Glucuronidase, Heparin-N-sulfatase, β-Hexosaminidase, Idunoratsulfatase, α-L-Idunoridase, α-Mannosidase, β-Mannosidase, Sialidase und Sphingomyelinase.

14. Samen nach einem der Ansprüche 9 bis 13, wobei der Samen von einer *Leguminose,* Getreide oder Tabak stammt.

15. Verfahren zur Herstellung des Samens nach einem der Ansprüche 9 bis 14, **dadurch gekennzeichnet, dass**:
- Pflanzenzellen durch die Verwendung eines Expressionsvektors transformiert werden, umfassend:
(a) einen Promotor eines Pflanzengens, welcher spezifisch ist für die Expression in Samenspeicherorganen und welcher stadiumspezifisch ist;
(b) eine DNA-Sequenz, welche die Signalsequenz eines Pflanzenproteins codiert, welches in der Lage ist, den Transport des lysosomalen Enzyms zu den Samenspeicherorganen zu vermitteln und die post-translationalen Modifizierungen bereitzustellen, welche für die Expression des Enzyms in aktiver Form erforderlich sind;
(c) eine DNA-Sequenz, welche das lysosomale Enzym ohne die native Signalsequenz codiert;
- die Zellen verwendet werden, um transformierte Pflanzen, welche in der Lage sind, Samen zu erzeugen, zu regenerieren.

16. Verfahren nach Anspruch 15, wobei der Samen von einer *Leguminose,* Getreide oder Tabak stammt.

17. Verfahren zum Extrahieren und Reinigen des lysosomalen Enzyms in aktiver Form, welches in den Samen nach einem der Ansprüche 9 bis 14 enthalten ist,
**dadurch gekennzeichnet, dass**
(a) der Samen in flüssigem Stickstoff in der Gegenwart eines Extraktionspuffers gemahlen wird;
(b) die resultierende Lösung zentrifugiert wird;
(c) der Überstand abgenommen und mit Filtern filtriert wird, welche eine Durchlässigkeit haben, die für die Größenordnung der Enzyme geeignet ist;
(d) das partiell gereinigte Enzym weiter durch HPLC-Chromatographie gereinigt wird.

18. Verwendung des Samens nach einem der Ansprüche 9 bis 14 zur Herstellung eines Medikaments für Enzymersatztherapien.

19. Verwendung des Samens nach Anspruch 18 zur Herstellung eines Medikaments für eine Enzymersatztherapie bei der Gaucher-Krankheit.

20. Verwendung des Samens nach Anspruch 18 zur Herstellung eines Medikaments für eine Enzymersatztherapie bei der Anderson-Fabry-Krankheit.

21. Verwendung des Samens nach Anspruch 18 zur Herstellung eines Medikaments für eine Enzymersatztherapie bei der Pompe-Krankheit.

22. Verwendung des Samens nach einem der Ansprüche 9 bis 14 als Mittel für die Speicherung und Erhaltung eines lysosomalen Enzyms in enzymatisch aktiver Form.

## Revendications

1. Végétal génétiquement transformé capable de produire une enzyme lysosomale d'origine humaine ou animale, **caractérisé en ce que** :
- ledit végétal est transformé via l'utilisation d'un vecteur d'expression comportant :
a. un promoteur d'un gène végétal spécifique à l'expression dans des organes de stockage de graine et spécifique à un stade,
b. une séquence d'ADN codant pour la séquence signal d'une protéine végétale capable de répartir ladite enzyme lysosomale dans des organes de stockage de graine et fournir les modifications post-translationnelles nécessaires à l'expression de l'enzyme sous une forme active,
c. une séquence d'ADN codant pour ladite enzyme lysosomale délétée de la séquence signal native,
- ladite enzyme est exprimée dans les tissus de stockage de graine sous une forme enzymatiquement active et selon une quantité d'au moins 0,8 % des protéines totales de la graine.

2. Végétal selon la revendication 1, **caractérisé en ce que** le vecteur d'expression est un plasmide.

3. Végétal selon la revendication 1 ou 2, **caractérisé en ce que** le promoteur est dérivé du gène de globuline 7S du soja.

4. Végétal selon l'une quelconque des revendications 1 à 3, **caractérisé en ce que** la séquence d'ADN codant pour la séquence signal est dérivée du gène de la globuline 7S du soja et est fusionnée à la séquence codant pour la partie structurelle de l'enzyme lysosomale mature délétée de la séquence signal native.

5. Végétal selon l'une quelconque des revendications 1 à 4, dans lequel l'enzyme lysosomale exprimée sous une forme enzymatiquement active dans des tissus de stockage de graine est :
α-N-acétylgalactosaminidase, lipase acide, arylsulfatase A, aspartylglycosaminidase, céramidase, α-fucosidase, α-galactosidase A, β-galactosidase, galactosylcéramidase, glucocérébrosidase, α-glucosidase, β-glucoronidase, héparine N-sulfatase, β-hexosaminidase, iduronate sulfatase, α-L-iduronidase, α-mannosidase, β-mannosidase, sialidase et sphingomyélinase.

6. Végétal selon l'une quelconque des revendications 1 à 5, dans lequel ledit végétal est *Leguminosa,* une céréale, ou du tabac.

7. Procédé pour produire le végétal génétiquement transformé capable de produire une enzyme lysosomale selon l'une quelconque des revendications 1 à 6, **caractérisé en ce que** :
- des cellules végétales sont transformées via l'utilisation d'un vecteur d'expression comportant :
a. un promoteur d'un gène végétal spécifique à l'expression dans des organes de stockage de graine et spécifique à un stade,
b. une séquence d'ADN codant pour la séquence signal d'une protéine végétale capable de répartir ladite enzyme lysosomale dans des organes de stockage de graine et fournir les modifications post-translationnelles nécessaires à l'expression de l'enzyme sous une forme active,
c. une séquence d'ADN codant pour ladite enzyme lysosomale délétée de la séquence signal native,
- lesdites cellules sont utilisées pour régénérer ledit végétal transformé.

8. Procédé selon la revendication 7, dans lequel ledit végétal est *Leguminosa,* une céréale, ou du tabac.

9. Graine d'un végétal génétiquement modifié capable d'exprimer une enzyme lysosomale, **caractérisée en ce que** :
- ladite graine contient un vecteur d'expression comportant :
a. un promoteur d'un gène végétal spécifique à l'expression dans des organes de stockage de graine et spécifique à un stade,
b. une séquence d'ADN codant pour la séquence signal d'une protéine végétale capable de répartir ladite enzyme lysosomale dans des organes de stockage de graine et fournir les modifications post-translationnelles nécessaires à l'expression de l'enzyme sous une forme active,
c. une séquence d'ADN codant pour ladite enzyme lysosomale délétée de la séquence signal native,
- ladite enzyme est contenue dans des tissus de stockage de graine sous une forme enzymatiquement active et selon la quantité d'au moins 0,8 % des protéines totales de graine.

10. Graine selon la revendication 8, **caractérisée en ce que** le vecteur d'expression est un plasmide.

11. Graine selon la revendication 9 ou 10, **caractérisée en ce que** le promoteur est dérivé du gène de globuline 7S du soja.

12. Graine selon l'une quelconque des revendications 9 à 11, **caractérisée en ce que** la séquence d'ADN codant pour la séquence signal est dérivée du gène de globuline 7S du soja et est fusionnée à la séquence codant pour la partie structurelle de l'enzyme lysosomale mature délétée de la séquence signal native.

13. Graine selon l'une quelconque des revendications 9 à 12, **caractérisée en ce que** l'enzyme lysosomale exprimée sous une forme enzymatiquement active dans des tissus de stockage de graine est :
α-N-acétylgalactosaminidase, lipase acide, arylsulfatase A, aspartylglycosaminidase, céramidase, α-fucosidase, α-galactosidase A, β-galactosidase, galactosylcéramidase, glucocérébrosidase, α-glucosidase, β-glucoronidase, héparine N-sulfatase, β-hexosaminidase, iduronate sulfatase, α-L-iduronidase, α-mannosidase, β-mannosidase, sialidase et sphingomyélinase.

14. Graine selon l'une quelconque des revendications 9 à 13, dans laquelle ladite graine est *Leguminosa,* une céréale, ou du tabac.

15. Procédé pour produire la graine selon l'une quelconque des revendications 9 à 14, **caractérisé en ce que** :
- des cellules végétales sont transformées via l'utilisation d'un vecteur d'expression comportant :
a. un promoteur d'un gène végétal spécifique à l'expression dans des organes de stockage de graine et spécifique à un stade,
b. une séquence d'ADN codant pour la séquence signal d'une protéine végétale capable de répartir ladite enzyme lysosomale dans des organes de stockage de graine et fournir les modifications post-translationnelles nécessaires à l'expression de l'enzyme sous une forme active,
c. une séquence d'ADN codant pour ladite enzyme lysosomale délétée de la séquence signal native,
- lesdites cellules sont utilisées pour régénérer des végétaux transformés capables de produire lesdites graines.

16. Procédé selon la revendication 15, dans lequel ladite graine est *Leguminosa,* une céréale, ou du tabac.

17. Procédé pour extraire et/ou purifier l'enzyme lysosomale sous une forme active contenue dans la graine selon l'une quelconque des revendications 9 à 14, **caractérisé en ce que** :
a. ladite graine est broyée dans de l'azote liquide en présence d'un tampon d'extraction,
b. la solution résultante est centrifugée,
c. le surnageant est récupéré et filtré à l'aide de filtres ayant une porosité adaptée aux dimensions d'enzyme,
d. l'enzyme partiellement purifiée est de plus purifiée par chromatographie HPLC.

18. Utilisation de la graine selon les revendications 9 à 14, pour la préparation de médicaments destinés à des enzymothérapies de remplacement.

19. Utilisation de la graine selon la revendication 18, pour la préparation d'un médicament destiné à une enzymothérapie de remplacement lors d'une maladie de Gaucher.

20. Utilisation de la graine selon la revendication 18, pour la préparation d'un médicament destiné à une enzymothérapie de remplacement lors d'une maladie de Anderson-Fabry.

21. Utilisation de la graine selon la revendication 18, pour la préparation d'un médicament destiné à une enzymothérapie de remplacement lors d'une maladie de Pompe.

22. Utilisation de la graine selon l'une quelconque des revendications 9 à 14, en tant que moyens pour stocker et préserver une enzyme lysosomale sous une forme enzymatiquement active.
